# EUROPEAN PATENT APPLICATION

(11) **EP 4 071 168 A1**
(43) Date of publication of application: **12.10.2022**
(21) Application number: 20896286.0
(22) Date of filing: 04.12.2020
(51) Int. Cl.: C07K 16/08, C12N 15/09, C12N 5/10, A61K 39/42

(54) **ANTIBODY AGAINST HUMAN CYTOMEGALOVIRUS AND USE THEREOF**

(30) Priority: 04.12.2019 CN 201911226892
(71) Applicant: Trinomab Biotech Co., Ltd., Zhuhai, Guangdong 519090 (CN)
(72) Inventor: LIAO, Huaxin, Zhuhai, Guangdong 519090 (CN); WANG, Yueming, Zhuhai, Guangdong 519090 (CN); WU, Changwen, Zhuhai, Guangdong 519090 (CN); ZHENG, Weihong, Zhuhai, Guangdong 519090 (CN)
(74) Representative: Elzaburu S.L.P.
(86) International application number: PCT/CN2020/133816
(87) International publication number: WO 2021/110126

(57) **Abstract**

Disclosed are a monoclonal antibody that is specific to human cytomegalovirus and binds to human cytomegalovirus with a high affinity, or an antigen-binding fragment thereof, and a method for preparing the antibody. The antibody is also highly effective in neutralizing infections. Also disclosed are an epitope to which the antibody binds, and the use of the antibody in the diagnosis, prevention and treatment of an infected individual.

SEQUENCE LISTING

<110>  Zhuhai Trinomab Biotechnology Co., Ltd.

<120>  antibody against human cytomegalovirus and use thereof

<130>  MTPIEP220125

<140>  PCT/CN2020/133816
<141>  2020-12-04

<150>  CN201911226892.6
<151>  2019-12-04

<160>  45

<170>  PatentIn version 3.3

<210>  1
<211>  8
<212>  PRT
<213>  Artificial Sequence

<220>
<223>  synthetic

<400>  1

Gly Phe Thr Phe Arg His Tyr Trp
1               5


<210>  2
<211>  8
<212>  PRT
<213>  Artificial Sequence

<220>
<223>  synthetic

<400>  2

Gly Phe Thr Phe Ser Asn His Gly
1               5


<210>  3
<211>  8
<212>  PRT
<213>  Artificial Sequence

<220>
<223>  synthetic

<400>  3

Gly Tyr Thr Phe Thr Asn Tyr Trp
1               5


<210>  4
<211>  8
<212>  PRT

<213>  Artificial Sequence

<220>
<223>  synthetic

<400>  4

Gly Phe Ser Phe Ser Thr Tyr Ala
1               5


<210>  5
<211>  8
<212>  PRT
<213>  Artificial Sequence

<220>
<223>  synthetic

<400>  5

Ile His Gly Ser Gly Thr Thr Thr
1               5


<210>  6
<211>  8
<212>  PRT
<213>  Artificial Sequence

<220>
<223>  synthetic

<400>  6

Ile Ser Ser Asp Gly Thr Asp Thr
1               5


<210>  7
<211>  8
<212>  PRT
<213>  Artificial Sequence

<220>
<223>  synthetic

<400>  7

Ile Phe Pro Arg Asp Ser Tyr Ser
1               5


<210>  8
<211>  8
<212>  PRT
<213>  Artificial Sequence

<220>
<223>  synthetic

<400>  8

Val Ser Gly Arg Gly Thr Ser Thr
1               5

<210>  9
<211>  10
<212>  PRT
<213>  Artificial Sequence

<220>
<223>  synthetic

<400>  9

Val Arg Asp Asp Tyr Thr Ser Gly Tyr Asn
1               5                   10

<210>  10
<211>  18
<212>  PRT
<213>  Artificial Sequence

<220>
<223>  synthetic

<400>  10

Ala Arg Asp Gly Arg Cys Gly Asp Glu Arg Cys Tyr Ser Gly Leu Pro
1               5                   10                  15

Asp Val

<210>  11
<211>  11
<212>  PRT
<213>  Artificial Sequence

<220>
<223>  synthetic

<400>  11

Ala Ile Tyr Asn Asp Leu Arg Ser Gly Asn Ser
1               5                   10

<210>  12
<211>  21
<212>  PRT
<213>  Artificial Sequence

<220>
<223>  synthetic

<400>  12

Ala Lys Asp Ser Tyr Val Gly Gly Ser Phe Asp Trp Leu Pro Arg Pro
1               5                   10                  15

Asp Tyr Phe Asp His
            20

<210>  13
<211>  117
<212>  PRT
<213>  Artificial Sequence

<220>
<223>  synthetic

<400>  13

Gln Val Leu Leu Val Gln Ser Gly Gly Gly Leu Val Gln Pro Gly Gly
1               5                   10                  15

Ser Leu Arg Leu Ala Cys Glu Thr Ser Gly Phe Thr Phe Arg His Tyr
            20                  25                  30

Trp Met His Trp Val Arg Gln Ala Pro Gly Lys Gly Leu Val Trp Leu
        35                  40                  45

Ser Ser Ile His Gly Ser Gly Thr Thr Thr Arg Tyr Ala Asp Ser Val
    50                  55                  60

Lys Gly Arg Phe Thr Ile Ser Arg Asp Asn Ala Lys Asn Ile Leu Phe
65                  70                  75                  80

Leu Gln Ile Asn Ser Pro Arg Pro Glu Asp Thr Ala Thr Tyr Tyr Cys
                85                  90                  95

Val Arg Asp Asp Tyr Thr Ser Gly Tyr Asn Trp Gly Gln Gly Ala Phe
            100                 105                 110

Val Thr Val Ser Ser
        115

<210>  14
<211>  125
<212>  PRT
<213>  Artificial Sequence

<220>
<223>  synthetic

<400>  14

Gln Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Asn His
            20                  25                  30

Gly Ile His Trp Val Arg Gln Ala Pro Gly Lys Gly Leu Glu Trp Val
        35                  40                  45

Ala Val Ile Ser Ser Asp Gly Thr Asp Thr Arg Asp Ala Asp Ser Val
    50                  55                  60

Arg Gly Arg Phe Thr Ile Ser Arg Asp Asn Ser Arg Asn Thr Val Tyr
65                  70                  75                  80

Leu Asp Met Asn Ser Leu Arg Ala Glu Asp Thr Ala Val Tyr Tyr Cys
                85                  90                  95

Ala Arg Asp Gly Arg Cys Gly Asp Glu Arg Cys Tyr Ser Gly Leu Pro
            100                 105                 110

Asp Val Trp Gly Arg Gly Thr Arg Val Ser Val Ser Ser
        115                 120                 125

<210>  15
<211>  118
<212>  PRT
<213>  Artificial Sequence

<220>
<223>  synthetic

<400>  15

Gln Val Gln Leu Val Gln Ser Gly Ala Glu Val Lys Lys Pro Gly Glu
1               5                   10                  15

Ser Leu Lys Ile Ser Cys Lys Gly Ser Gly Tyr Thr Phe Thr Asn Tyr
            20                  25                  30

Trp Ile Gly Trp Val Arg Gln Met Pro Gly Ala Gly Leu Glu Trp Met
        35                  40                  45

Ala Ile Ile Phe Pro Arg Asp Ser Tyr Ser Ala Tyr Ser Pro Ser Phe
    50                  55                  60

Gln Gly Arg Val Thr Ile Ser Val Asp Lys Ser Ile Ser Thr Ala Tyr
65                  70                  75                  80

Leu His Trp Ser Ser Leu Glu Ala Ser Asp Thr Ala Val Tyr Tyr Cys
                85                  90                  95

Ala Ile Tyr Asn Asp Leu Arg Ser Gly Asn Ser Trp Gly Gln Gly Thr
            100                 105                 110

Pro Leu Ile Val Ser Ser
        115

<210>  16
<211>  128
<212>  PRT
<213>  Artificial Sequence

<220>
<223>  synthetic

<400>  16

Glu Val Gln Leu Val Glu Ser Gly Gly Gly Leu Ala Gln Pro Gly Gly
1               5                   10                  15

Ser Leu Arg Leu Ser Cys Glu Ala Ser Gly Phe Ser Phe Ser Thr Tyr
            20                  25                  30

Ala Met Ser Trp Val Arg Gln Ala Pro Gly Lys Gly Leu Glu Trp Val
        35                  40                  45

Ala Ala Val Ser Gly Arg Gly Thr Ser Thr Phe Tyr Leu Asp Ser Val
    50                  55                  60

Lys Gly Arg Phe Thr Val Ser Arg Asp Asn Ser Arg Thr Thr Val Phe
65                  70                  75                  80

Leu Gln Met Asn Ser Leu Lys Val Glu Asp Thr Ala Phe Tyr Tyr Cys
                85                  90                  95

Ala Lys Asp Ser Tyr Val Gly Gly Ser Phe Asp Trp Leu Pro Arg Pro
            100                 105                 110

Asp Tyr Phe Asp His Trp Gly Arg Gly Val Leu Val Thr Val Ser Ser
        115                 120                 125

<210>  17
<211>  106
<212>  PRT
<213>  Artificial Sequence

<220>
<223>  synthetic

<400>  17

Gly Gln Pro Lys Ala Ala Pro Ser Val Thr Leu Phe Pro Pro Ser Ser
1               5                   10                  15

Glu Glu Leu Gln Ala Asn Lys Ala Thr Leu Val Cys Leu Ile Ser Asp
            20                  25                  30

Phe Tyr Pro Gly Ala Val Thr Val Ala Trp Lys Ala Asp Ser Ser Pro
        35                  40                  45

Val Lys Ala Gly Val Glu Thr Thr Thr Pro Ser Lys Gln Ser Asn Asn
    50                  55                  60

Lys Tyr Ala Ala Ser Ser Tyr Leu Ser Leu Thr Pro Glu Gln Trp Lys
65                  70                  75                  80

Ser His Arg Ser Tyr Ser Cys Gln Val Thr His Glu Gly Ser Thr Val
                85                  90                  95

Glu Lys Thr Val Ala Pro Thr Glu Cys Ser
            100                 105

<210>  18
<211>  107
<212>  PRT
<213>  Artificial Sequence

<220>
<223>  synthetic

<400>  18

<210>  19
<211>  731
<212>  PRT

<213>  Artificial Sequence

<220>
<223>  gB_Towne strain

<400>  19

Met Glu Ser Arg Ile Trp Cys Leu Val Val Cys Val Asn Leu Cys Ile
1               5                   10                  15

Val Cys Leu Gly Ala Ala Val Ser Ser Ser Ser Thr Arg Gly Thr Ser
            20                  25                  30

Ala Thr His Ser His His Ser Ser His Thr Thr Ser Ala Ala His Ser
        35                  40                  45

Arg Ser Gly Ser Val Ser Gln Arg Val Thr Ser Ser Gln Thr Val Ser
    50                  55                  60

His Gly Val Asn Glu Thr Ile Tyr Asn Thr Thr Leu Lys Tyr Gly Asp
65                  70                  75                  80

Val Val Gly Val Asn Thr Thr Lys Tyr Pro Tyr Arg Val Cys Ser Met
                85                  90                  95

Ala Gln Gly Thr Asp Leu Ile Arg Phe Glu Arg Asn Ile Val Cys Thr
            100                 105                 110

Ser Met Lys Pro Ile Asn Glu Asp Leu Asp Glu Gly Ile Met Val Val
        115                 120                 125

Tyr Lys Arg Asn Ile Val Ala His Thr Phe Lys Val Arg Val Tyr Gln
    130                 135                 140

Lys Val Leu Thr Phe Arg Arg Ser Tyr Ala Tyr Ile His Thr Thr Tyr
145                 150                 155                 160

Leu Leu Gly Ser Asn Thr Glu Tyr Val Ala Pro Pro Met Trp Glu Ile
                165                 170                 175

His His Ile Asn Ser His Ser Gln Cys Tyr Ser Ser Tyr Ser Arg Val
            180                 185                 190

Ile Ala Gly Thr Val Phe Val Ala Tyr His Arg Asp Ser Tyr Glu Asn
        195                 200                 205

Lys Thr Met Gln Leu Met Pro Asp Asp Tyr Ser Asn Thr His Ser Thr
    210                 215                 220

Arg Tyr Val Thr Val Lys Asp Gln Trp His Ser Arg Gly Ser Thr Trp
225                 230                 235                 240

Leu Tyr Arg Glu Thr Cys Asn Leu Asn Cys Met Val Thr Ile Thr Thr
                245                 250                 255

Ala Arg Ser Lys Tyr Pro Tyr His Phe Phe Ala Thr Ser Thr Gly Asp
            260                 265                 270

Val Val Asp Ile Ser Pro Phe Tyr Asn Gly Thr Asn Arg Asn Ala Ser
        275                 280                 285

Tyr Phe Gly Glu Asn Ala Asp Lys Phe Phe Ile Phe Pro Asn Tyr Thr
    290                 295                 300

Ile Val Ser Asp Phe Gly Arg Pro Asn Ser Ala Leu Glu Thr His Arg
305                 310                 315                 320

Leu Val Ala Phe Leu Glu Arg Ala Asp Ser Val Ile Ser Trp Asp Ile
                325                 330                 335

Gln Asp Glu Lys Asn Val Thr Cys Gln Leu Thr Phe Trp Glu Ala Ser
            340                 345                 350

Glu Arg Thr Ile Arg Ser Glu Ala Glu Asp Ser Tyr His Phe Ser Ser
        355                 360                 365

Ala Lys Met Thr Ala Thr Phe Leu Ser Lys Lys Gln Glu Val Asn Met
    370                 375                 380

Ser Asp Ser Ala Leu Asp Cys Val Arg Asp Glu Ala Ile Asn Lys Leu
385                 390                 395                 400

Gln Gln Ile Phe Asn Thr Ser Tyr Asn Gln Thr Tyr Glu Lys Tyr Gly
                405                 410                 415

Asn Val Ser Val Phe Glu Thr Thr Gly Gly Leu Val Val Phe Trp Gln
            420                 425                 430

Gly Ile Lys Gln Lys Ser Leu Val Glu Leu Glu Arg Leu Ala Asn Arg
        435                 440                 445

Ser Ser Leu Asn Leu Thr His Asn Glu Thr Lys Glu Ser Thr Asp Gly
    450                 455                 460

Asn Asn Ala Thr His Leu Ser Asn Met Glu Ser Val His Asn Leu Val
465                 470                 475                 480

Tyr Ala Gln Leu Gln Phe Thr Tyr Asp Thr Leu Arg Gly Tyr Ile Asn
                485                 490                 495

Arg Ala Leu Ala Gln Ile Ala Glu Ala Trp Cys Val Asp Gln Arg Arg
            500                 505                 510

Thr Leu Glu Val Phe Lys Glu Leu Ser Lys Ile Asn Pro Ser Ala Ile
        515                 520                 525

Leu Ser Ala Ile Tyr Asn Lys Pro Ile Ala Ala Arg Phe Met Gly Asp
    530                 535                 540

Val Leu Gly Leu Ala Ser Cys Val Thr Ile Asn Gln Thr Ser Val Lys
545                 550                 555                 560

Val Leu Arg Asp Met Asn Val Lys Glu Ser Pro Gly Arg Cys Tyr Ser
                565                 570                 575

Arg Pro Val Val Ile Phe Asn Phe Ala Asn Ser Ser Tyr Val Gln Tyr
            580                 585                 590

Gly Gln Leu Gly Glu Asp Asn Glu Ile Leu Leu Gly Asn His Arg Thr
        595                 600                 605

Glu Glu Cys Gln Leu Pro Ser Leu Lys Ile Phe Ile Ala Gly Asn Ser
    610                 615                 620

Ala Tyr Glu Tyr Val Asp Tyr Leu Phe Lys Arg Met Ile Asp Leu Ser
625                 630                 635                 640

Ser Ile Ser Thr Val Asp Ser Met Ile Ala Leu Asp Ile Asp Pro Leu
                645                 650                 655

Glu Asn Thr Asp Phe Arg Val Leu Glu Leu Tyr Ser Gln Lys Glu Leu
            660                 665                 670

Arg Ser Ile Asn Val Phe Asp Leu Glu Glu Ile Met Arg Glu Phe Asn
        675                 680                 685

Ser Tyr Lys Gln Arg Val Lys Tyr Val Glu Asp Lys Gly Leu Asn Asp
    690                 695                 700

Ile Phe Glu Ala Gln Lys Ile Glu Trp His Glu Leu Glu Val Leu Phe
705                 710                 715                 720

Gln Gly Pro Gly His His His His His His His
                725                 730

<210>  20
<211>  729
<212>  PRT
<213>  Artificial Sequence

<220>
<223>  gB_Con. strain

<400>  20

Met Glu Ser Arg Ile Trp Cys Leu Val Val Cys Val Asn Leu Cys Ile
1               5                   10                  15

Val Cys Leu Gly Ala Ala Val Ser Ser Ser Ser Thr Ser His Ala Thr
            20                  25                  30

Ser Ser Thr His Asn Gly Ser His Thr Ser Arg Thr Thr Ser Ala Gln
        35                  40                  45

Thr Arg Ser Val Ser Gln His Val Thr Ser Ser Glu Ala Val Ser His
    50                  55                  60

Arg Ala Asn Glu Thr Ile Tyr Asn Thr Thr Leu Lys Tyr Gly Asp Val
65                  70                  75                  80

Val Gly Val Asn Thr Thr Lys Tyr Pro Tyr Arg Val Cys Ser Met Ala
                85                  90                  95

Gln Gly Thr Asp Leu Ile Arg Phe Glu Arg Asn Ile Val Cys Thr Pro
            100                 105                 110

Met Lys Pro Ile Asn Glu Asp Leu Asp Glu Gly Ile Met Val Val Tyr
        115                 120                 125

Lys Arg Asn Ile Val Ala His Thr Phe Lys Val Arg Val Tyr Gln Lys
    130                 135                 140

Val Leu Thr Phe Arg Arg Ser Tyr Ala Tyr Ile His Thr Thr Tyr Leu
145                 150                 155                 160

Leu Gly Ser Asn Thr Glu Tyr Val Ala Pro Pro Met Trp Glu Ile His
                165                 170                 175

His Ile Asn Ser His Ser Gln Cys Tyr Ser Ser Tyr Ser Arg Val Ile
            180                 185                 190

Ala Gly Thr Val Phe Val Ala Tyr His Arg Asp Ser Tyr Glu Asn Lys
        195                 200                 205

Thr Met Gln Leu Met Pro Asp Asp Tyr Ser Asn Thr His Ser Thr Arg
    210                 215                 220

Tyr Val Thr Val Lys Asp Gln Trp His Ser Arg Gly Ser Thr Trp Leu
225                 230                 235                 240

Tyr Arg Glu Thr Cys Asn Leu Asn Cys Met Val Thr Ile Thr Thr Ala
                245                 250                 255

Arg Ser Lys Tyr Pro Tyr His Phe Phe Ala Thr Ser Thr Gly Asp Val
            260                 265                 270

Val Asp Ile Ser Pro Phe Tyr Asn Gly Thr Asn Arg Asn Ala Ser Tyr
        275                 280                 285

Phe Gly Glu Asn Ala Asp Lys Phe Phe Ile Phe Pro Asn Tyr Thr Ile
    290                 295                 300

Val Ser Asp Phe Gly Arg Pro Asn Ser Ala Pro Glu Thr His Arg Leu
305                 310                 315                 320

Val Ala Phe Leu Glu Arg Ala Asp Ser Val Ile Ser Trp Asp Ile Gln
                325                 330                 335

Asp Glu Lys Asn Val Thr Cys Gln Leu Thr Phe Trp Glu Ala Ser Glu
            340                 345                 350

Arg Thr Ile Arg Ser Glu Ala Glu Asp Ser Tyr His Phe Ser Ser Ala
        355                 360                 365

Lys Met Thr Ala Thr Phe Leu Ser Lys Lys Gln Glu Val Asn Met Ser
    370                 375                 380

Asp Ser Ala Leu Asp Cys Val Arg Asp Glu Ala Ile Asn Lys Leu Gln
385                 390                 395                 400

Gln Ile Phe Asn Thr Ser Tyr Asn Gln Thr Tyr Glu Lys Tyr Gly Asn
                405                 410                 415

Val Ser Val Phe Glu Thr Thr Gly Gly Leu Val Val Phe Trp Gln Gly
            420                 425                 430

Ile Lys Gln Lys Ser Leu Val Glu Leu Glu Arg Leu Ala Asn Arg Ser
        435                 440                 445

Ser Leu Asn Leu Thr His Glu Thr Lys Glu Ser Thr Asp Gly Asn Asn
    450                 455                 460

Thr Thr His Leu Ser Asn Met Glu Ser Val His Asn Leu Val Tyr Ala
465                 470                 475                 480

Gln Leu Gln Phe Thr Tyr Asp Thr Leu Arg Gly Tyr Ile Asn Arg Ala
                485                 490                 495

Leu Ala Gln Ile Ala Glu Ala Trp Cys Val Asp Gln Arg Arg Thr Leu
            500                 505                 510

Glu Val Phe Lys Glu Leu Ser Lys Ile Asn Pro Ser Ala Ile Leu Ser
        515                 520                 525

Ala Ile Tyr Asn Lys Pro Ile Ala Ala Arg Phe Met Gly Asp Val Leu
    530                 535                 540

Gly Leu Ala Ser Cys Val Thr Ile Asn Gln Thr Ser Val Lys Val Leu
545                 550                 555                 560

Arg Asp Met Asn Val Lys Glu Ser Pro Gly Arg Cys Tyr Ser Arg Pro
                565                 570                 575

Val Val Ile Phe Asn Phe Ala Asn Ser Ser Tyr Val Gln Tyr Gly Gln
            580                 585                 590

Leu Gly Glu Asp Asn Glu Ile Leu Leu Gly Asn His Arg Thr Glu Glu
        595                 600                 605

Cys Gln Leu Pro Ser Leu Lys Ile Phe Ile Ala Gly Asn Ser Ala Tyr
    610                 615                 620

Glu Tyr Val Asp Tyr Leu Phe Lys Arg Met Ile Asp Leu Ser Ser Ile
625                 630                 635                 640

Ser Thr Val Asp Ser Met Ile Ala Leu Asp Ile Asp Pro Leu Glu Asn
                645                 650                 655

Thr Asp Phe Arg Val Leu Glu Leu Tyr Ser Gln Lys Glu Leu Arg Ser
            660                 665                 670

Ser Asn Val Phe Asp Leu Glu Glu Ile Met Arg Glu Phe Asn Ser Tyr
        675                 680                 685

Lys Gln Arg Val Lys Tyr Val Glu Asp Lys Gly Leu Asn Asp Ile Phe
    690                 695                 700

Glu Ala Gln Lys Ile Glu Trp His Glu Leu Glu Val Leu Phe Gln Gly

705                 710                 715                 720

Pro Gly His His His His His His His
                725

<210>  21
<211>  9
<212>  PRT
<213>  Artificial Sequence

<220>
<223>  synthetic

<400>  21

Arg Ser Asp Val Gly Gly Tyr Asn Tyr
1               5

<210>  22
<211>  6
<212>  PRT
<213>  Artificial Sequence

<220>
<223>  synthetic

<400>  22

Gln Ser Val Gly Gly Tyr
1               5

<210>  23
<211>  6
<212>  PRT
<213>  Artificial Sequence

<220>
<223>  synthetic

<400>  23

Gln Ser Ile Thr Lys Tyr
1               5

<210>  24
<211>  8
<212>  PRT
<213>  Artificial Sequence

<220>
<223>  synthetic

<400>  24

Asn Ser Asn Ile Gly Lys Asn Tyr
1               5

<210>  25
<211>  3
<212>  PRT
<213>  Artificial Sequence

<220>
<223>  synthetic

<400>  25

Asp Val Ser
1


<210>  26
<211>  3
<212>  PRT
<213>  Artificial Sequence

<220>
<223>  synthetic

<400>  26

Asp Ala Ser
1


<210>  27
<211>  3
<212>  PRT
<213>  Artificial Sequence

<220>
<223>  synthetic

<400>  27

Thr Thr Ser
1


<210>  28
<211>  3
<212>  PRT
<213>  Artificial Sequence

<220>
<223>  synthetic

<400>  28

Asn Asn Asn
1


<210>  29
<211>  11
<212>  PRT
<213>  Artificial Sequence

<220>
<223>  synthetic

<400>  29

Ser Ser Tyr Thr Thr Lys Ser Thr Leu Tyr Val
1               5                   10


<210>  30
<211>  10
<212>  PRT
<213>  Artificial Sequence

<220>
<223>  synthetic

<400>  30

Gln Gln Arg Ser Asn Trp Pro Pro Leu Thr
1               5                   10


<210>  31
<211>  9
<212>  PRT
<213>  Artificial Sequence

<220>
<223>  synthetic

<400>  31

Gln Gln Ser Phe Ser Thr Leu Trp Thr
1               5


<210>  32
<211>  11
<212>  PRT
<213>  Artificial Sequence

<220>
<223>  synthetic

<400>  32

Ala Thr Trp Asp Lys Thr Leu Asn Phe Trp Val
1               5                   10


<210>  33
<211>  111
<212>  PRT
<213>  Artificial Sequence

<220>
<223>  synthetic

<400>  33

Gln Ser Ala Leu Thr Gln Pro Ala Ser Val Ser Gly Ser Arg Gly Gln
1               5                   10                  15

Ser Ile Thr Ile Pro Cys Thr Gly Ser Arg Ser Asp Val Gly Gly Tyr
                20                  25                  30

Asn Tyr Val Ser Trp Tyr Gln Gln Arg Pro Gly Lys Ala Pro Lys Leu
        35                  40                  45

Ile Leu Tyr Asp Val Ser His Arg Pro Ser Gly Ile Ser Asp Arg Phe
    50                  55                  60

Ser Gly Ser Lys Leu Gly Asp Thr Ala Ser Leu Thr Ile Ser Gly Leu
65                  70                  75                  80

Arg Asp Glu Asp Glu Ala Asp Tyr Tyr Cys Ser Ser Tyr Thr Thr Lys
                85                  90                  95

Ser Thr Leu Tyr Val Phe Gly Thr Gly Thr Thr Val Ser Val Leu
                100                 105                 110

<210>  34
<211>  108
<212>  PRT
<213>  Artificial Sequence

<220>
<223>  synthetic

<400>  34

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Gly Tyr
                20                  25                  30

Leu Ala Trp Tyr Gln Gln Lys Val Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Pro
                85                  90                  95

Leu Thr Phe Gly Gly Gly Thr Lys Val Glu Ile Lys
                100                 105

<210>  35

<211>  108
<212>  PRT
<213>  Artificial Sequence

<220>
<223>  synthetic


<220>
<221>  misc_feature
<222>  (108)..(108)
<223>  Xaa can be any naturally occurring amino acid

<400>  35

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Thr Lys Tyr
            20                  25                  30


Leu Asn Trp Tyr Gln Gln Lys Pro Gly Arg Ala Pro Lys Leu Leu Ile
        35                  40                  45


His Thr Thr Ser Thr Leu Gln Ser Gly Val Pro Ser Arg Phe Ser Gly
    50                  55                  60


Ser Gly Ser Gly Thr Asp Phe Thr Leu Thr Ile Ser Ser Leu Gln Leu
65                  70                  75                  80


Glu Asp Phe Gly Thr Tyr Tyr Cys Gln Gln Ser Phe Ser Thr Leu Trp
                85                  90                  95


Thr Phe Gly Gln Gly Thr Lys Leu Asp Ile Lys Xaa
            100                 105


<210>  36
<211>  109
<212>  PRT
<213>  Artificial Sequence

<220>
<223>  synthetic

<400>  36

Gln Ser Val Leu Thr Gln Pro Pro Ser Leu Ser Ala Thr Pro Gly Gln
1               5                   10                  15


Thr Val Thr Ile Ser Cys Ser Gly Ser Asn Ser Asn Ile Gly Lys Asn
            20                  25                  30


Tyr Val Ser Trp Tyr Gln Gln Leu Pro Gly Ala Ala Pro Lys Leu Leu
        35                  40                  45

Leu Tyr Asn Asn Asn Leu Arg Pro Ser Gly Ile Pro Ala Arg Phe Ser
    50                  55                  60

Gly Ser Lys Ser Gly Thr Ser Ala Thr Leu Gly Ile Thr Gly Leu Gln
65                  70                  75                  80

Ala Gly Asp Glu Ala Thr Tyr Ser Cys Ala Thr Trp Asp Lys Thr Leu
                85                  90                  95

Asn Phe Trp Val Phe Gly Gly Gly Thr Lys Val Thr Val
            100                 105

<210>  37
<211>  447
<212>  PRT
<213>  Artificial Sequence

<220>
<223>  synthetic

<400>  37

Gln Val Leu Leu Val Gln Ser Gly Gly Gly Leu Val Gln Pro Gly Gly
1               5                   10                  15

Ser Leu Arg Leu Ala Cys Glu Thr Ser Gly Phe Thr Phe Arg His Tyr
            20                  25                  30

Trp Met His Trp Val Arg Gln Ala Pro Gly Lys Gly Leu Val Trp Leu
        35                  40                  45

Ser Ser Ile His Gly Ser Gly Thr Thr Thr Arg Tyr Ala Asp Ser Val
    50                  55                  60

Lys Gly Arg Phe Thr Ile Ser Arg Asp Asn Ala Lys Asn Ile Leu Phe
65                  70                  75                  80

Leu Gln Ile Asn Ser Pro Arg Pro Glu Asp Thr Ala Thr Tyr Tyr Cys
                85                  90                  95

Val Arg Asp Asp Tyr Thr Ser Gly Tyr Asn Trp Gly Gln Gly Ala Phe
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Arg Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210> 38
<211> 217
<212> PRT
<213> Artificial Sequence

<220>
<223> synthetic

<400> 38

Gln Ser Ala Leu Thr Gln Pro Ala Ser Val Ser Gly Ser Arg Gly Gln
1               5                   10                  15

Ser Ile Thr Ile Pro Cys Thr Gly Ser Arg Ser Asp Val Gly Gly Tyr
            20                  25                  30

Asn Tyr Val Ser Trp Tyr Gln Gln Arg Pro Gly Lys Ala Pro Lys Leu
        35                  40                  45

Ile Leu Tyr Asp Val Ser His Arg Pro Ser Gly Ile Ser Asp Arg Phe
    50                  55                  60

Ser Gly Ser Lys Leu Gly Asp Thr Ala Ser Leu Thr Ile Ser Gly Leu
65                  70                  75                  80

Arg Asp Glu Asp Glu Ala Asp Tyr Tyr Cys Ser Ser Tyr Thr Thr Lys
                85                  90                  95

Ser Thr Leu Tyr Val Phe Gly Thr Gly Thr Thr Val Ser Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  39
<211>  455
<212>  PRT
<213>  Artificial Sequence

<220>
<223>  synthetic

<400>  39

Gln Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Asn His
            20                  25                  30

Gly Ile His Trp Val Arg Gln Ala Pro Gly Lys Gly Leu Glu Trp Val
        35                  40                  45

Ala Val Ile Ser Ser Asp Gly Thr Asp Thr Arg Asp Ala Asp Ser Val
    50                  55                  60

Arg Gly Arg Phe Thr Ile Ser Arg Asp Asn Ser Arg Asn Thr Val Tyr
65                  70                  75                  80

Leu Asp Met Asn Ser Leu Arg Ala Glu Asp Thr Ala Val Tyr Tyr Cys
                85                  90                  95

Ala Arg Asp Gly Arg Cys Gly Asp Glu Arg Cys Tyr Ser Gly Leu Pro
            100                 105                 110

Asp Val Trp Gly Arg Gly Thr Arg Val Ser Val Ser Ser Ala Ser Thr
        115                 120                 125

Lys Gly Pro Ser Val Phe Pro Leu Ala Pro Ser Ser Lys Ser Thr Ser
    130                 135                 140

Gly Gly Thr Ala Ala Leu Gly Cys Leu Val Lys Asp Tyr Phe Pro Glu
145                 150                 155                 160

Pro Val Thr Val Ser Trp Asn Ser Gly Ala Leu Thr Ser Gly Val His
                165                 170                 175

Thr Phe Pro Ala Val Leu Gln Ser Ser Gly Leu Tyr Ser Leu Ser Ser
            180                 185                 190

Val Val Thr Val Pro Ser Ser Ser Leu Gly Thr Gln Thr Tyr Ile Cys
        195                 200                 205

Asn Val Asn His Lys Pro Ser Asn Thr Lys Val Asp Lys Arg Val Glu
    210                 215                 220

Pro Lys Ser Cys Asp Lys Thr His Thr Cys Pro Pro Cys Pro Ala Pro
225                 230                 235                 240

Glu Leu Leu Gly Gly Pro Ser Val Phe Leu Phe Pro Pro Lys Pro Lys
                245                 250                 255

Asp Thr Leu Met Ile Ser Arg Thr Pro Glu Val Thr Cys Val Val Val
            260                 265                 270

Asp Val Ser His Glu Asp Pro Glu Val Lys Phe Asn Trp Tyr Val Asp
        275                 280                 285

Gly Val Glu Val His Asn Ala Lys Thr Lys Pro Arg Glu Glu Gln Tyr
    290                 295                 300

Asn Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Ala Leu
                325                 330                 335

Pro Ala Pro Ile Glu Lys Thr Ile Ser Lys Ala Lys Gly Gln Pro Arg
            340                 345                 350

Glu Pro Gln Val Tyr Thr Leu Pro Pro Ser Arg Glu Glu Met Thr Lys
        355                 360                 365

Asn Gln Val Ser Leu Thr Cys Leu Val Lys Gly Phe Tyr Pro Ser Asp
    370                 375                 380

Ile Ala Val Glu Trp Glu Ser Asn Gly Gln Pro Glu Asn Asn Tyr Lys
385                 390                 395                 400

Thr Thr Pro Pro Val Leu Asp Ser Asp Gly Ser Phe Phe Leu Tyr Ser
                405                 410                 415

Lys Leu Thr Val Asp Lys Ser Arg Trp Gln Gln Gly Asn Val Phe Ser
            420                 425                 430

Cys Ser Val Met His Glu Ala Leu His Asn His Tyr Thr Gln Lys Ser
        435                 440                 445

Leu Ser Leu Ser Pro Gly Lys
    450                 455

<210>  40
<211>  215
<212>  PRT
<213>  Artificial Sequence

<220>
<223>  synthetic

<400>  40

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Gly Gly Tyr
            20                  25                  30

Leu Ala Trp Tyr Gln Gln Lys Val Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Pro
                85                  90                  95

Leu Thr Phe Gly Gly Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  41
<211>  448
<212>  PRT
<213>  Artificial Sequence

<220>
<223>  synthetic

<400>  41

Gln Val Gln Leu Val Gln Ser Gly Ala Glu Val Lys Lys Pro Gly Glu
1               5                   10                  15

Ser Leu Lys Ile Ser Cys Lys Gly Ser Gly Tyr Thr Phe Thr Asn Tyr
            20                  25                  30

Trp Ile Gly Trp Val Arg Gln Met Pro Gly Ala Gly Leu Glu Trp Met
        35                  40                  45

Ala Ile Ile Phe Pro Arg Asp Ser Tyr Ser Ala Tyr Ser Pro Ser Phe
    50                  55                  60

Gln Gly Arg Val Thr Ile Ser Val Asp Lys Ser Ile Ser Thr Ala Tyr
65                  70                  75                  80

Leu His Trp Ser Ser Leu Glu Ala Ser Asp Thr Ala Val Tyr Tyr Cys
                85                  90                  95

Ala Ile Tyr Asn Asp Leu Arg Ser Gly Asn Ser Trp Gly Gln Gly Thr
            100                 105                 110

Pro Leu Ile Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly

    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Arg Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  42
<211>  214
<212>  PRT
<213>  Artificial Sequence

<220>
<223>  synthetic

<400>  42

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Thr Lys Tyr
            20                  25                  30

Leu Asn Trp Tyr Gln Gln Lys Pro Gly Arg Ala Pro Lys Leu Leu Ile
        35                  40                  45

His Thr Thr Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Leu
65                  70                  75                  80

Glu Asp Phe Gly Thr Tyr Tyr Cys Gln Gln Ser Phe Ser Thr Leu Trp
                85                  90                  95

Thr Phe Gly Gln Gly Thr Lys Leu Asp Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln

145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  43
<211>  458
<212>  PRT
<213>  Artificial Sequence

<220>
<223>  synthetic

<400>  43

Glu Val Gln Leu Val Glu Ser Gly Gly Gly Leu Ala Gln Pro Gly Gly
1               5                   10                  15

Ser Leu Arg Leu Ser Cys Glu Ala Ser Gly Phe Ser Phe Ser Thr Tyr
            20                  25                  30

Ala Met Ser Trp Val Arg Gln Ala Pro Gly Lys Gly Leu Glu Trp Val
        35                  40                  45

Ala Ala Val Ser Gly Arg Gly Thr Ser Thr Phe Tyr Leu Asp Ser Val
    50                  55                  60

Lys Gly Arg Phe Thr Val Ser Arg Asp Asn Ser Arg Thr Thr Val Phe
65                  70                  75                  80

Leu Gln Met Asn Ser Leu Lys Val Glu Asp Thr Ala Phe Tyr Tyr Cys
                85                  90                  95

Ala Lys Asp Ser Tyr Val Gly Gly Ser Phe Asp Trp Leu Pro Arg Pro
            100                 105                 110

Asp Tyr Phe Asp His Trp Gly Arg Gly Val Leu Val Thr Val Ser Ser
        115                 120                 125

Ala Ser Thr Lys Gly Pro Ser Val Phe Pro Leu Ala Pro Ser Ser Lys
    130                 135                 140

Ser Thr Ser Gly Gly Thr Ala Ala Leu Gly Cys Leu Val Lys Asp Tyr
145                 150                 155                 160

Phe Pro Glu Pro Val Thr Val Ser Trp Asn Ser Gly Ala Leu Thr Ser
                165                 170                 175

Gly Val His Thr Phe Pro Ala Val Leu Gln Ser Ser Gly Leu Tyr Ser
            180                 185                 190

Leu Ser Ser Val Val Thr Val Pro Ser Ser Ser Leu Gly Thr Gln Thr
        195                 200                 205

Tyr Ile Cys Asn Val Asn His Lys Pro Ser Asn Thr Lys Val Asp Lys
    210                 215                 220

Arg Val Glu Pro Lys Ser Cys Asp Lys Thr His Thr Cys Pro Pro Cys
225                 230                 235                 240

Pro Ala Pro Glu Leu Leu Gly Gly Pro Ser Val Phe Leu Phe Pro Pro
                245                 250                 255

Lys Pro Lys Asp Thr Leu Met Ile Ser Arg Thr Pro Glu Val Thr Cys
            260                 265                 270

Val Val Val Asp Val Ser His Glu Asp Pro Glu Val Lys Phe Asn Trp
        275                 280                 285

Tyr Val Asp Gly Val Glu Val His Asn Ala Lys Thr Lys Pro Arg Glu
    290                 295                 300

Glu Gln Tyr Asn Ser Thr Tyr Arg Val Val Ser Val Leu Thr Val Leu
305                 310                 315                 320

His Gln Asp Trp Leu Asn Gly Lys Glu Tyr Lys Cys Lys Val Ser Asn
                325                 330                 335

Lys Ala Leu Pro Ala Pro Ile Glu Lys Thr Ile Ser Lys Ala Lys Gly
            340                 345                 350

Gln Pro Arg Glu Pro Gln Val Tyr Thr Leu Pro Pro Ser Arg Glu Glu
        355                 360                 365

Met Thr Lys Asn Gln Val Ser Leu Thr Cys Leu Val Lys Gly Phe Tyr
    370                 375                 380

Pro Ser Asp Ile Ala Val Glu Trp Glu Ser Asn Gly Gln Pro Glu Asn

385                 390                 395                 400

Asn Tyr Lys Thr Thr Pro Pro Val Leu Asp Ser Asp Gly Ser Phe Phe
            405                 410                 415

Leu Tyr Ser Lys Leu Thr Val Asp Lys Ser Arg Trp Gln Gln Gly Asn
        420                 425                 430

Val Phe Ser Cys Ser Val Met His Glu Ala Leu His Asn His Tyr Thr
    435                 440                 445

Gln Lys Ser Leu Ser Leu Ser Pro Gly Lys
    450                 455

<210>  44
<211>  216
<212>  PRT
<213>  Artificial Sequence

<220>
<223>  synthetic

<400>  44

Gln Ser Val Leu Thr Gln Pro Pro Ser Leu Ser Ala Thr Pro Gly Gln
1               5                   10                  15

Thr Val Thr Ile Ser Cys Ser Gly Ser Asn Ser Asn Ile Gly Lys Asn
            20                  25                  30

Tyr Val Ser Trp Tyr Gln Gln Leu Pro Gly Ala Ala Pro Lys Leu Leu
        35                  40                  45

Leu Tyr Asn Asn Asn Leu Arg Pro Ser Gly Ile Pro Ala Arg Phe Ser
    50                  55                  60

Gly Ser Lys Ser Gly Thr Ser Ala Thr Leu Gly Ile Thr Gly Leu Gln
65                  70                  75                  80

Ala Gly Asp Glu Ala Thr Tyr Ser Cys Ala Thr Trp Asp Lys Thr Leu
                85                  90                  95

Asn Phe Trp Val Phe Gly Gly Gly Thr Lys Val Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  45
<211>  330
<212>  PRT
<213>  Artificial Sequence

<220>
<223>  synthetic

<400>  45

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

## Description

The present invention relates to antibodies or antigen-binding fragments thereof which are specific to human cytomegalovirus and bind thereto with high affinity, and a method for producing the same. The antibodies of the present invention also have high potency to neutralize infection. The present invention also relates to epitopes to which the antibodies bind, and to the use of the antibodies in the diagnosis, prevention and treatment of infected individuals.

### Background Art

The human cytomegalovirus (HCMV) is a widely distributed pathogen with an infection rate of 40%-100% in the population, which causes serious complications and even death in those with compromised immune function, such as organ transplant recipients, bone marrow transplant recipients, HIV patients, pregnant women and fetuses, and is involved in malignant tumors and chronic diseases. In the United States and Europe, the incidence of live births infected with HCMV is estimated to be 0.2%-1.2%; the infection rate of women of childbearing age in China is as high as 95.3%, and the intrauterine infection rate caused by the primary infection in pregnant women is about 30.0%-40.0%. HCMV seropositivity is more prevalent in developing countries or low-income regions.

After primary infection, the HCMV can be latent, undergo productive viral replication, allow the virus to spread in the host, and evade the immune system of the host, and it is difficult to completely eliminate HCMV. In general, most people infected with HCMV are asymptomatic. In people with illness and compromised immune function (e.g., transplant recipients, HIV patients, etc.), primary infection or reactivation of HCMV may result in lesions of the urinary system, central nervous system, liver, lung, blood circulatory system, etc.; HCMV also has secondary immunosuppression and increases the chance of infection, leading to morbidity and mortality. In addition, HCMV is a major cause of infection in birth defects, including organ defects, developmental delay, mental retardation, and visual and auditory loss, caused by vertical transmission through pregnant women. There is currently no therapy to prevent or treat fetal infection. Prevymis (MSD) for the prevention of HCMV infection and related diseases is the first new HCMV infection drug approved by FDA in the United States in the past 15 years and is only applicable to adult patients. Cytogam and Cytotect are two marketed preparations of cytomegalovirus immunoglobulin, but show clinically significant side effects and uncertainty of therapeutic efficacy.

Because of the extremely important role of antibodies in immune defense, especially the fact that antibodies are indispensable for anti-infection, despite numerous failures of anti-human cytomegalovirus monoclonal antibodies, there have been a reason to choose them for further research. In light of the current advances in antibody technology, it is more likely that monoclonal antibodies with high neutralizing capacity obtained from human B cells are more potent.

The antibodies disclosed in the present invention are derived from human B cells, which remedies the deficiencies of the prior art, and the present invention aims to provide a fully human monoclonal antibody with strong specificity and high neutralizing titer, which is suitable for the medical treatment, prevention and/or diagnosis of HCMV infection, and can also be used for the treatment of various disorders associated with HCMV infection.

### Summary of the Invention

The present invention thus provides a novel antibody that binds to human cytomegalovirus and antigen-binding fragments thereof, as well as novel epitopes to which the antibody binds.

In one aspect, the present invention provides a highly specific antibody or antigen-binding fragment thereof that neutralizes human cytomegalovirus infection at high titers. In some embodiments, the antibody or antigen-binding fragment thereof of the present invention binds to the envelope glycoprotein gB of human cytomegalovirus.

In some embodiments, the present invention includes an antibody or antigen-binding fragment thereof, which is capable of specifically binding to the human cytomegalovirus gB glycoprotein fusion domain.

In some embodiments, the present invention includes an antibody or antigen-binding fragment thereof, which binds to an epitope on the gB glycoprotein fusion domain comprising one or more amino acid residues selected from the group consisting of N208, L213, and Y226 of the gB glycoprotein fusion domain. In some embodiments, an antibody or antigen-binding fragment thereof of the present invention binds to an epitope on the gB glycoprotein fusion domain comprising amino acid residues L213 and Y226 of the gB glycoprotein fusion domain. In some embodiments, an antibody or antigen-binding fragment thereof of the present invention binds to an epitope on the gB glycoprotein fusion domain comprising amino acid residue N208 of the gB glycoprotein fusion domain.

In another aspect, the present invention provides a highly specific antibody or antigen-binding fragment thereof that neutralizes HCMV infection at high titers. In some embodiments, the anti-human cytomegalovirus antibody or antigen-binding fragment thereof of the present invention comprises at least one, at least two or all three of the three complementarity determining regions (CDRs) contained in a heavy chain variable region (VH).

In some embodiments, the anti-human cytomegalovirus antibody or antigen-binding fragment thereof of the present invention comprises at least one, at least two or all three of the three complementarity determining regions (CDRs) contained in a light chain variable region (VH).

In some embodiments, the anti-human cytomegalovirus antibody or antigen-binding fragment thereof of the present invention comprises a heavy chain variable region (VH), wherein the VH comprises one, two, or three complementarity determining regions (CDRs) shown in SEQ ID NO: 13, 14, 15, or 16.

In some embodiments, the anti-human cytomegalovirus antibody or antigen-binding fragment thereof of the present invention comprises a light chain variable region (VL), wherein the VL comprises one, two, or three complementarity determining regions (CDRs) shown in SEQ ID NO: 33, 34, 35, or 36.

In some embodiments, the anti-human cytomegalovirus antibody or antigen-binding fragment thereof of the present invention comprises a heavy chain variable region (VH) and a light chain variable region (VL), wherein
(a) the VH comprises three complementarity determining regions (CDRs) shown in SEQ ID NO: 13, 14, 15, or 16; and/or
(b) the VL comprises three complementarity determining regions (CDRs) shown in SEQ ID NO: 33, 34, 35, or 36.

In a preferred embodiment, VH comprises or consists of an amino acid sequence selected from the group consisting of SEQ ID NO 13, 14, 15, or 16.

In a preferred embodiment, VL comprises or consists of an amino acid sequence selected from the group consisting of SEQ ID NO 33, 34, 35, or 36.

In a preferred embodiment, the anti-human cytomegalovirus antibody or antigen-binding fragment thereof of the present invention comprises
three heavy chain complementarity determining regions (HCDRs) of the heavy chain variable region shown in SEQ ID NO: 13, 14, 15, or 16, and the three light chain complementarity determining regions (LCDRs) of the light chain variable region shown in SEQ ID NO: 33, 34, 35, or 36.

In some embodiments, the anti-human cytomegalovirus antibody or antigen-binding fragment thereof of the present invention comprises 1, 2, 3, 4, 5, or 6 of the exemplified combinations of HCDR1, HCDR2, HCDR3, LCDR1, LCDR2, and LCDR3 of the antibodies or antigen-binding fragments thereof of the present invention shown in Table A.

In some embodiments, the anti-human cytomegalovirus antibody or antigen-binding fragment thereof of the present invention comprises HCDR3 comprising or consisting of the amino acid sequence of SEQ ID NO: 9, 10, 11 or 12.

In some embodiments, the anti-human cytomegalovirus antibody or antigen-binding fragment thereof of the present invention comprises a heavy chain variable region (VH) and/or a light chain variable region (VL), wherein
(i) the VH comprises the complementarity determining regions HCDR1, HCDR2, and HCDR3, wherein HCDR1 comprises or consists of the amino acid sequence of SEQ IDNO: 1, 2, 3, or 4; HCDR2 comprises or consists of the amino acid sequence of SEQ ID NO: 5, 6, 7, or 8; HCDR3 comprises or consists of the amino acid sequence of SEQ ID NO: 9, 10, 11, or 12;
   and/or
(ii) wherein the VL comprises the complementarity determining regions LCDR1, LCDR2, and LCDR3, wherein LCDR1 comprises or consists of the amino acid sequence of SEQ ID NO: 21, 22, 23, or 24; LCDR2 comprises or consists of the amino acid sequence of SEQ ID NO: 25, 26, 27, or 28; LCDR3 comprises or consists of an amino acid sequence selected from the group consisting of SEQ ID NO: 29, 30, 31, or 32.

In a preferred embodiment, the present invention provides an anti-human cytomegalovirus antibody or antigen-binding fragment thereof, comprising a heavy chain variable region (VH) and a light chain variable region (VL), wherein
(a) the VH comprises
   (i) HCDR1, HCDR2, and HCDR3 comprising or consisting of the sequences of SEQ ID NO: 1, SEQ ID NO: 5, and SEQ ID NO: 9; or
   (ii) HCDR1, HCDR2, and HCDR3 comprising or consisting of the sequences of SEQ ID NO: 2, SEQ ID NO: 6, and SEQ ID NO: 10; or
   (iii) HCDR1, HCDR2, and HCDR3 comprising or consisting of the sequences of SEQ ID NO: 3, SEQ ID NO: 7, and SEQ ID NO: 11; or
   (iv) HCDR1, HCDR2, and HCDR3 comprising or consisting of the sequences of SEQ ID NO: 4, SEQ ID NO: 8, and SEQ ID NO: 12; or
      and/or
(b) the VL comprises
   (i) LCDR1, LCDR2, and LCDR3 comprising or consisting of the sequences of SEQ ID NO: 21, SEQ ID NO: 25, and SEQ ID NO: 29; or
   (ii) LCDR1, LCDR2, and LCDR3 comprising or consisting of the sequences of SEQ ID NO: 22, SEQ ID NO: 26, and SEQ ID NO: 30; or
   (iii) LCDR1, LCDR2, and LCDR3 comprising or consisting of the sequences of SEQ ID NO: 23, SEQ ID NO: 27, and SEQ ID NO: 31; or
   (iv) LCDR1, LCDR2, and LCDR3 comprising or consisting of the sequences of SEQ ID NO: 24, SEQ ID NO: 28, and SEQ ID NO: 32.

In a preferred embodiment, the present invention provides an anti-human cytomegalovirus antibody or antigen-binding fragment thereof, comprising a heavy chain variable region (VH) and a light chain variable region (VL), wherein the VH comprises the complementarity determining regions HCDR1, HCDR2, and HCDR3; and the VL comprises complementarity determining regions LCDR1, LCDR2, and LCDR3, wherein the combination of HCDR1, HCDR2, HCDR3, LCDR1, LCDR2, and LCDR3 contained in the antibody or antigen-binding fragment thereof is shown in the following table (Table A):

**Table A: Exemplary combinations of HCDRI, HCDR2, HCDR3, LCDR1, LCDR2, and LCDR3 in the antibody or antigen-binding fragment thereof of the present invention.**

| Combination | HCDR1 comprising or consisting of the amino acid sequence shown in the following SEQ ID NOs | HCDR2 comprising or consisting of the amino acid sequence shown in the following SEQ ID NOs | HCDR3 comprising or consisting of the amino acid sequence shown in the following SEQ ID NOs | LCDR1 comprising or consisting of the amino acid sequences shown in the following SEQ ID NOs | LCDR2 comprising or consisting of the amino acid sequence shown in the following SEQ ID NOs | LCDR3 comprising or consisting of the amino acid sequences shown in the following SEQ ID NOs |
|---|---|---|---|---|---|---|
| (1) | SEQ ID NO: 1 | SEQ ID NO: 5 | SEQ ID NO: 9 | SEQ ID NO: 21 | SEQ ID NO: 25 | SEQ ID NO: 29 |
| (2) | SEQ ID NO: 2 | SEQ ID NO: 6 | SEQ ID NO: 10 | SEQ ID NO: 22 | SEQ ID NO: 26 | SEQ ID NO: 30 |
| (3) | SEQ ID NO: 3 | SEQ ID NO: 7 | SEQ ID NO: 11 | SEQ ID NO: 23 | SEQ ID NO: 27 | SEQ ID NO: 31 |
| (4) | SEQ ID NO: 4 | SEQ ID NO: 8 | SEQ ID NO: 12 | SEQ ID NO: 24 | SEQ ID NO: 28 | SEQ ID NO: 32 |

In some embodiments, the anti-human cytomegalovirus antibody or antigen-binding fragment thereof of the present invention comprises a heavy chain variable region VH and/or a light chain variable region VL, wherein
(a) the heavy chain variable region VH
   (i) comprises or consists of an amino acid sequence that is at least 90%, 91%, 92%, 93%, 94%, 95%, 96%, 97%, 98%, or 99% identical to an amino acid sequence selected from the group consisting of SEQ ID NOs: 13, 14, 15, or 16; or
   (ii) comprises or consists of an amino acid sequence selected from SEQ ID NO: 13, 14, 15, or 16; or
   (iii) comprises an amino acid sequence having 1 or more (preferably no more than 10, more preferably no more than 5, 4, 3, 2, 1) amino acid changes (preferably amino acid substitutions, more preferably amino acid conservative substitutions) compared to an amino acid sequence selected from SEQ ID NO 13, 14, 15 or 16, preferably the amino acid changes do not occur in the CDR regions;
      and/or
(b) the light chain variable region VL
   (i) comprises or consists of an amino acid sequence that is at least 90%, 91%, 92%, 93%, 94%, 95%, 96%, 97%, 98%, or 99% identical to an amino acid sequence selected from the group consisting of SEQ ID NO: 33, 34, 35, or 36; or
   (ii) comprises or consists of an amino acid sequence selected from SEQ ID NO: 33, 34, 35, or 36; or
   (iii) comprises an amino acid sequence having 1 or more (preferably no more than 10, more preferably no more than 5, 4, 3, 2, 1) amino acid changes (preferably amino acid substitutions, more preferably amino acid conservative substitutions) compared to an amino acid sequence selected from SEQ ID NO: 33, 34, 35 or 36, preferably the amino acid changes do not occur in the CDR regions.

In a preferred embodiment, the present invention provides an anti-human cytomegalovirus antibody or antigen-binding fragment thereof, comprising a heavy chain variable region (VH) and a light chain variable region (VL), wherein the antibody or antigen-binding fragment thereof comprises a combination of a heavy chain variable region VH and a light chain variable region VL as shown in the following Table (Table B):

**Table B: Exemplary combinations of a heavy chain variable region VH and a light chain variable region VL in the antibody or antigen-binding fragment thereof of the present invention.**

| Combination | VH comprising or consisting of the amino acid sequence shown in the following SEQ ID NOs | VL comprising or consisting of the amino acid sequence shown in the following SEQ ID NOs |
|---|---|---|
| (1) | SEQ ID NO: 13 | SEQ ID NO: 33 |
| (2) | SEQ ID NO: 14 | SEQ ID NO: 34 |
| (3) | SEQ ID NO: 15 | SEQ ID NO: 35 |
| (4) | SEQ ID NO: 16 | SEQ ID NO: 36 |

In some embodiments, the antibodies of the present invention are in the form of IgG, IgM, IgA, IgD, or IgE. In some embodiments, the antibodies of the present invention comprise a heavy chain constant region selected from, for example, the heavy chain constant regions of IgG, IgM, IgA, IgD, or IgE; in particular, a heavy chain constant region selected from the heavy chain constant region of IgG, more specifically from the heavy chain constant region of IgG1, IgG2, IgG3, or IgG4, e.g. from the heavy chain constant region of human IgG1, IgG2, IgG3, or IgG 4. In one embodiment, the heavy chain constant region is a human IgG1 or human IgG3 heavy chain constant region. In one embodiment, the heavy chain constant region is a human IgG2 or human IgG4 heavy chain constant region. In one embodiment, the heavy chain constant region is a human IgG1 heavy chain constant region. In another embodiment, the antibody molecule of the present invention has a light chain constant region, e.g., selected from κ or λ light chain constant region, preferably from a light chain constant region of κ (e.g., human κ).

In yet another embodiment, the antibody molecule of the present invention comprises the heavy chain constant region of IgG1 (e. g., human IgG1). In yet another embodiment, the antibody molecule of the present invention comprises the heavy chain constant region of IgG3 (e. g., human IgG3). In one embodiment, the heavy chain constant region comprises or consists of the amino acid sequence shown in SEQ ID NO: 45, or a sequence having at least 80%, 85%, 90%, 91%, 92%, 93%, 94%, 95%, 96%, 97%, 98%, 99% or more identity thereto.

In yet another embodiment, the antibody molecule of the present invention comprises a κ light chain constant region, e.g., a human κ light chain constant region. In one embodiment, the light chain constant region comprises or consists of the amino acid sequence shown in SEQ ID NO: 18, or a sequence having at least 80%, 85%, 90%, 91%, 92%, 93%, 94%, 95%, 96%, 97%, 98%, 99% or more identity thereto.

In yet another embodiment, the antibody molecule of the present invention comprises a λ light chain constant region, e.g., a human λ light chain constant region. In one embodiment, the light chain constant region comprises or consists of the amino acid sequence shown in SEQ ID NO: 17, or a sequence having at least 80%, 85%, 90%, 91%, 92%, 93%, 94%, 95%, 96%, 97%, 98%, 99% or more identity thereto.

In some embodiments, the anti-human cytomegalovirus antibody or antigen-binding fragment thereof of the present invention comprises a heavy chain and/or a light chain, wherein
(a) the heavy chain
   (i) comprises or consists of an amino acid sequence that is at least 85%, 90%, 91%, 92%, 93%, 94%, 95%, 96%, 97%, 98%, or 99% identical to an amino acid sequence selected from the group consisting of SEQ ID NOs: 37, 39, 41, or 43; or
   (ii) comprises or consists of an amino acid sequence selected from SEQ ID NO: 37, 39, 41, or 43; or
   (iii) comprises an amino acid sequence having 1 or more (preferably no more than 20 or 10, more preferably no more than 5, 4, 3, 2, 1) amino acid changes(preferably amino acid substitutions, more preferably amino acid conservative substitutions) compared to an amino acid sequence selected from SEQ ID NO 37, 39, 41 or 43, preferably the amino acid changes do not occur in the CDR regions of the heavy chain, more preferably the amino acid changes do not occur in the variable region of the heavy chain;
      and/or
(b) the light chain
   (i) comprises or consists of an amino acid sequence that is at least 85%, 90%, 91%, 92%, 93%, 94%, 95%, 96%, 97%, 98%, or 99% identical to an amino acid sequence selected from the group consisting of SEQ ID NOs: 38, 40, 42, or 44; or
   (ii) comprises or consists of an amino acid sequence selected from SEQ ID NO: 38, 40, 42, or 44; or
   (iii) comprises an amino acid sequence having 1 or more (preferably no more than 20 or 10, more preferably no more than 5, 4, 3, 2, 1) amino acid changes (preferably amino acid substitutions, more preferably amino acid conservative substitutions) compared to an amino acid sequence selected from SEQ ID NO 38, 40, 42 or 44, preferably the amino acid changes do not occur in the CDR regions of the light chain, more preferably the amino acid changes do not occur in the variable region of the light chain.

In a preferred embodiment, the present invention provides an anti-human cytomegalovirus antibody or antigen-binding fragment thereof, comprising a heavy chain and a light chain, wherein the antibody or antigen-binding fragment thereof comprises a combination of heavy and light chains shown in the following table (Table C):

**Table C: Exemplary combinations of heavy and light chains in the antibody or antigen-binding fragment thereof of the present invention**

| Combination | Heavy chain comprising or consisting of the amino acid sequence shown in the following SEQ ID NOs | Light chain comprising or consisting of the amino acid sequence shown in the following SEQ ID NOs |
|---|---|---|
| (1) | SEQ ID NO: 37 | SEQ ID NO: 38 |
| (2) | SEQ ID NO: 39 | SEQ ID NO: 40 |
| (3) | SEQ ID NO: 41 | SEQ ID NO: 42 |
| (4) | SEQ ID NO: 43 | SEQ ID NO: 44 |

In one embodiment of the present invention, the amino acid changes described herein include substitutions, insertions or deletions of amino acids. Preferably, the amino acid changes described herein are amino acid substitutions, preferably conservative substitutions.

In preferred embodiments, the amino acid changes described herein occur in regions outside of CDR (e.g., in FR). More preferably, the amino acid changes described herein occur in regions outside the heavy chain variable region and/or outside the light chain variable region.

In some embodiments, the substitutions are conservative substitutions. A conservative substitution refers to a substitution of one amino acid for another within the same class, e.g., a substitution of one acidic amino acid for another acidic amino acid, a substitution of one basic amino acid for another basic amino acid, or a substitution of one neutral amino acid for another neutral amino acid. Exemplary substitutions are shown in table D below:

**Table D**

| Original residue | Exemplary substitution | Preferred conservative amino acid substitutions |
|---|---|---|
| Ala(A) | Val, Leu, Ile | Val |
| Arg(R) | Lys, Gln, Asn | Lys |
| Asn(N) | Gln, His, Asp, Lys, Arg | Gln |
| Asp(D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Asn, Gln, Lys, Arg | Arg |
| Ile (I) | Leu, Val, Met, Ala, Phe, Norleucine | Leu |
| Leu (L) | Norleucine, Ile, Val, Met, Ala, Phe | Ile |
| Lys (K) | Arg, Gln, Asn | Arg |
| Met (M) | Leu, Phe, Ile | Leu |
| Phe (F) | Trp, Leu, Val, Ile, Ala, Tyr | Tyr |
| Pro (P) | Ala | Ala |
| Ser (S) | Thr | Thr |
| Thr (T) | Val, Ser | Ser |
| Trp (W) | Tyr, Phe | Tyr |
| Tyr (Y) | Trp, Phe, Thr, Ser | Phe |
| Val (V) | Ile, Leu, Met, Phe, Ala, Norleucine | Leu |

In certain embodiments, the substitution occurs in the CDR region of the antibody. Typically, the resulting variant will have modifications (e.g., improvements) in certain biological properties (e.g., increased affinity) relative to the parent antibody and/or will have certain biological properties that are substantially retained by the parent antibody. An exemplary displacement variant is an affinity matured antibody.

In certain embodiments, the antibodies provided herein can be further modified to contain other non-protein moieties known and readily available in the art. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymers, carboxymethyl cellulose, dextran, polyvinyl alcohol, polyvinyl pyrrolidone, poly-1,3-dioxane, poly-1,3,6-trioxane, ethylene/maleic anhydride copolymers, poly amino acids (homopolymers or random copolymers), and dextran or poly (n-vinyl pyrrolidone polyethylene) glycol, propylene glycol homopolymers, poly propylene oxide/ethylene oxide copolymers, poly oxyethylated polyols (e.g., glycerol), polyvinyl alcohol, and mixtures thereof. For example, the antibody or antigen-binding fragment thereof of the present invention can be chemically modified by covalent coupling to a polymer, e.g., to increase its circulating half-life.

In certain embodiments, the antibodies provided herein are altered to increase or decrease the extent to which the antibody is glycosylated. The addition or deletion of a glycosylation site to an antibody can be conveniently accomplished by altering the amino acid sequence to create or remove one or more glycosylation sites. When an antibody comprises an Fc region, the carbohydrate attached thereto can be altered. Modifications that remove unwanted glycosylation sites may be useful in some applications, such as removal of the fucose module to enhance antibody-dependent cellular cytotoxicity (ADCC) function (see Shield et.al, (2002) JBC277:26733). In other applications, galactosylation modifications can be made to modify complement-dependent cytotoxicity (CDC).

In certain embodiments, it may be desirable to produce cysteine engineered antibodies, such as "thio MAb", in which one or more residues of the antibody are replaced with a cysteine residue. Cysteine engineered antibodies can be generated as described, for example, in U.S. Patent No.7,521,541.

In some embodiments, the anti-human cytomegalovirus antibodies or antigen-binding fragments thereof of the present invention exhibit the same or similar binding affinity and/or specificity to HCMV as an antibody of the present invention; and/or inhibit (e.g., competitively inhibit) the binding of the antibodies of the present invention to HCMV and/or bind to the same or overlapping epitopes as the antibodies of the present invention; and/or compete with the antibody of the present invention for binding to HCMV; and/or have one or more of the biological properties of the antibodies of the present invention.

In some embodiments, the antibodies or fragments thereof of the present invention are capable of efficiently neutralizing HCMV. In some embodiments, the antibodies of the present invention neutralize HCMV with an EC50 as low as 0.15 µg/mL or less. In some embodiments, the antibodies of the present invention neutralize HCMV with an EC50 of 0.15, 0.14, 0.13, 0.12, 0.11, 0.10, 0.09, 0.08, 0.07, 0.06, 0.05, 0.04, 0.03, and 0.02 µg/mL or less. In some embodiments, the antibodies of the present invention neutralize HCMV with a very low EC50 in endothelial cells or fibroblasts.

In some embodiments, the antibodies or antigen-binding fragments thereof of the present invention are capable of directly blocking virus invasion of host cells.

In some embodiments, the antibody of the present invention is capable of effectively binding to gB glycoprotein, e.g. binding to gB glycoprotein with an equilibrium dissociation constant (K_{D}) of less than or equal to about 50 nM, preferably less than or equal to about 40 nM, more preferably less than or equal to about 30 nM, more preferably less than or equal to about 20 nM, and most preferably less than or equal to about 5 nM, 4 nM, 3 nM, 2 nM, 1.5 nM, 1 nM, 0.9 nM, 0.8 nM, or 0.7 nM. In some embodiments, the antibody binding affinity is determined using a surface plasmon resonance (SPR) assay.

In some embodiments, at least a portion of the framework sequences of the antibody or antigen-binding fragment thereof of the present invention are human consensus framework sequences.

In some embodiments, the anti-human cytomegalovirus antibody of the present invention is an antibody in the IgG form, for example an antibody in the IgG1 form or an antibody in the IgG2 form or an antibody in the IgG3 form or an antibody in the IgG4 form.

In some embodiments, the anti-human cytomegalovirus antibody is a monoclonal antibody. In some embodiments, the anti-human cytomegalovirus antibody is humanized. In some embodiments, the anti-human cytomegalovirus antibody is a human antibody. In one embodiment, the anti-human cytomegalovirus antibody of the present invention also encompasses antibody fragments thereof, preferably antibody fragments selected from the group consisting of: Fab, Fab', Fab'-SH, Fv, single chain antibody (e.g., scFv) or (Fab')₂, single domain antibody, diabodies (dAb) or linear antibody.

In certain embodiments, the anti-human cytomegalovirus antibody molecule is in the form of a bispecific or multispecific antibody molecule.

In some embodiments, the antibodies of the present invention do not elicit an autoimmune response.

In some embodiments, the present invention also encompasses antibodies conjugated to a label ("immunoconjugates").

In some embodiments, the antibody of the present invention can be conjugated to a detectable label to facilitate the imaging of a site comprising cells of interest (e.g., a cell infected with HCMV). Labeled antibodies using a wide variety of labels can be used in a wide variety of assays. Detection of an antibody-antigen complex formed between an antibody of the present invention and an epitope of interest (HCMV epitope) is facilitated by attaching a detectable substance to the antibody. Suitable detection methods include the use of labels such as radionuclides, enzymes, coenzymes, fluorescers, chemiluminescent substances, chromogens, enzyme substrates or cofactors, enzyme inhibitors, prosthetic group complexes, free radicals, particles, dyes, and the like. Labeled antibodies can be used in a variety of well-known assays, such as radioimmunoassays, enzyme immunoassays (e.g., ELISA), and fluorescent immunoassays. Suitable labels and related methods are described, for example, in [15] US 3,766,162, US 3,791,932, US 3,817,837 or US 4,233,402, etc.

In some embodiments, the present invention provides nucleic acids encoding any of the antibodies or fragments thereof described herein, or any chain thereof. In one embodiment, a vector comprising the nucleic acid is provided. In one embodiment, the vector is an expression vector, such as a eukaryotic vector. In one embodiment, a host cell comprising the nucleic acid or the vector is provided.

For example, a nucleic acid of the present invention comprises:
a nucleic acid encoding an amino acid sequence selected from the group consisting of SEQ ID NOs: 13-16 and 33-44, or a nucleic acid encoding an amino acid sequence that is at least 85%, 90%, 91%, 92%, 93%, 94%, 95%, 96%, 97%, 98%, or 99% identical to an amino acid sequence selected from the group consisting of SEQ ID NOs: 13-16 and 33-44.

The present invention also encompasses nucleic acids that hybridize under stringent conditions to, or have one or more substitutions (e.g., conservative substitutions), deletions, or insertions with, nucleic acids described below: nucleic acids comprising a nucleic acid sequence encoding an amino acid sequence selected from any one of SEQ ID NOs: 13-16 and 33-44; or nucleic acids comprising a nucleic acid sequence encoding an amino acid sequence having at least 85%, 90%, 91%, 92%, 93%, 94%, 95%, 96%, 97%, 98% or 99% identity to an amino acid sequence selected from the group consisting of any one of SEQ ID NO: 13-16 and 33-44.

In one embodiment, one or more vectors comprising the nucleic acid are provided. In one embodiment, the vector is an expression vector, such as a eukaryotic expression vector. In some embodiments, the vector comprises an EF promoter and/or a CMV enhancer. For example, the vector includes, but is not limited to, viruses, plasmids, cosmids, λphage or yeast artificial chromosome (YAC). In one embodiment, for example, a pCDNA 3.1 vector is provided. Once the expression vector or DNA sequence has been prepared for expression, the expression vector may be transfected or introduced into a suitable host cell. Various techniques can be used to accomplish this purpose, for example, protoplast fusion, calcium phosphate precipitation, electroporation, retroviral transduction, viral transfection, gene gun, lipid-based transfection, or other conventional techniques. In the case of protoplast fusion, the cells are incubated in culture and screened for appropriate activity. Methods and conditions for culturing the resulting transfected cells and for recovering the resulting antibody molecules are known to those skilled in the art and may be varied or optimized based on the specification and methods known in the art, depending on the used particular expression vectors and mammalian host cells. Alternatively, cells that have stably incorporated DNA into their chromosomes can be selected by introducing one or more markers that allow selection of transfected host cells. The marker may, for example, provide prototrophy, biocidal resistance (e.g., antibiotics) or heavy metal (e.g., copper) resistance, etc.to an auxotrophic host. The selectable marker gene may be linked directly to the DNA sequence to be expressed or be introduced into the same cell by co-transformation. Additional elements may also be required in order to optimally synthesize mRNA. These elements may include splicing signals, as well as transcriptional promoters, enhancers, and termination signals.

In one embodiment, a host cell comprising one or more polynucleotides of the present invention is provided. In some embodiments, a host cell comprising an expression vector of the present invention is provided. In some embodiments, the host cell is preferably a cell commonly used for the expression of protein genes and the like, particularly suitable for the expression of antibody genes. Suitable host cells for the present invention include prokaryotic microorganisms, such as E. Coli. The host cell can also be a eukaryotic microorganism such as a filamentous fungus or yeast, or a variety of eukaryotic cells, e.g., insect cells or plant cells, etc. Vertebrate cells may also be used as hosts. For example, mammalian cell lines engineered to be suitable for growth in suspension may be used. Examples of useful mammalian host cell lines include the SV40 transformed monkey kidney CV1 line (COS-7); human embryonic kidney line (HEK293 or 293F cells), 293 cells, baby hamster kidney cells (BHK), monkey kidney cells (CV1), African green monkey kidney cells (VERO-76), human cervical cancer cells (HELA), canine kidney cells (MDCK), buffalo rat liver cells (BRL 3A), human lung cells (W138), human liver cells (Hep G2), Chinese hamster ovary cells (CHO cells), CHOK1SV cells, CHOK1SV GS-KO cells, CHOS cells, NSO cells, myeloma cell lines such as YO, NSO, P3X63, and Sp2/0, etc. For a review on mammalian host cell lines suitable for protein production see, e.g., Yazaki and Wu, Methods in Molecular Biology, vol. 248 (B. K. C. Lo, ed. Humana Press, Totowa, NJ), p. 255-268 (2003). In a preferred embodiment, the host cell is a CHO cell, such as a CHOS cell, CHOK1SV cell or CHOK1SV GS-KO, or the host cell is a 293 cell, such as a HEK293 cell.

In one embodiment, the host cell is a eukaryotic cell. In another embodiment, the host cell is selected from a yeast cell, a mammalian cell (e.g., a CHO cell or a 293 cell, such as a HEK293 cell), or other cells suitable for making the respective antibody or antigen-binding fragment thereof. In one embodiment, the host cell is a prokaryotic cell.

In some embodiments, host cells are, for example, E. Coli cells, actinomycete cells, yeast cells, insect cells (SF9, etc.), mammalian cells (293, HEK293, COS-1, CHO, myeloma cells, etc.).

Generally, a recombinant animal cell line such as a CHO cell line which stably produces the antibody at a high level is used to produce recombinant antibodies industrially. The production, cloning, and gene amplification for high expression and screening of such recombinant cell lines can be carried out by known methods (for example, see Omasa T: J. Biosci. Bioeng. 94, 600-605, 2002, etc.).

In one embodiment, a method of preparing an antibody molecule of the present invention is provided, wherein the method comprises culturing a host cell comprising a nucleic acid encoding the antibody (e.g., any one of the polypeptide chains and/or plurality of polypeptide chains) or an expression vector comprising the nucleic acid under conditions suitable for expression of the antibody, and optionally recovering the antibody from the host cell (or host cell culture medium or supernatant). To recombinantly produce an antibody molecule of the present invention, a nucleic acid encoding an antibody (e.g., an antibody described above, e.g., any one of the polypeptide chains and/or plurality of polypeptide chains) is isolated and inserted into one or more vectors for further cloning and/or expression in a host cell. Such nucleic acids are readily isolated and sequenced using conventional protocols (e. g., by using oligonucleotide probes capable of binding specifically to genes encoding the heavy and light chains of antibodies).

In a preferred embodiment of the present invention, the antibody of the present invention is a fully humanized antibody. A recombinant human antibody can be obtained using a known method (referring to Nature, 312: 643, 1984, Nature, 321: 522, 1986, etc.). For example, the antibody of the present invention can be produced by culturing a host cell into which the vector of the present invention is introduced and purifying the produced antibody from the culture supernatant, etc. More specifically, the antibody can be produced by the following method: the human antibody expression vector is constructed by inserting cDNA encoding VH and VL into an expression vector for animal cells containing genes encoding human antibody CH and/or human antibody CL produced by the same cell or different human cells, respectively, and introduced into the animal cells for expression. In some embodiments, expression vectors recombined with nucleic acids encoding VH or VL are typically prepared separately and co-transfected into host cells, but may also be recombined into a single expression vector.

In some embodiments, the polyclonal antibody or the monoclonal antibody can be obtained from experimental animals such as mice, rabbits, goats, etc.

In some embodiments, the anti-human cytomegalovirus monoclonal antibody or the antigen binding fragment thereof of the present invention can be obtained from cells produced by human blood-derived antibodies, and is a fully humanized antibody. The fully humanized antibody is not immunogenic and does not show an immune response even when administered to a human body as an antibody drug. For example, the present monoclonal antibodies and antigen-binding fragments thereof can be obtained by the following methods: a cell clone producing the antibody is isolated from the blood of a healthy person through various steps, the antibody is obtained from the culture supernatant of the cell clone, and the resulting antibody is subjected to affinity purification (see, e.g., WO2010/114106).

Antibody molecules prepared as described herein can be purified by known prior art techniques such as high performance liquid chromatography, ion exchange chromatography, gel electrophoresis, affinity chromatography, and size exclusion chromatography, etc. The actual conditions used to purify a particular protein will also depend on factors such as net charge, hydrophobicity, and hydrophilicity, and will be apparent to those skilled in the art. The purity of the antibody molecules of the present invention can be determined by any of a variety of well-known analytical methods, including size exclusion chromatography, gel electrophoresis, and high performance liquid chromatography, etc.

In some embodiments, the present invention also provides methods for identifying, screening, or characterizing the physical/chemical properties and/or biological activity of the antibody molecules of the present invention.

In one aspect, the antigen binding activity of the antibodies in the present invention are tested, e.g., by known methods such as ELISA, western blotting, etc. The binding to HCMV can be determined using methods known in the art, and exemplary methods are disclosed herein. In some embodiments, surface plasmon resonance assays (e.g., affinity measurements) or ELISA assays are used.

It will be appreciated that any of the above assays can be performed using the immunoconjugates of the present invention in place of or in addition to anti-human cytomegalovirus antibodies.

It will be appreciated that any of the above assays can be performed using a combination of anti-human cytomegalovirus antibodies and another active agents.

In some embodiments, the present invention provides a pharmaceutical composition comprising the antibodies of the present invention.

In some embodiments, the present invention provides a composition, preferably a pharmaceutical composition, comprising any of the antibody molecules described herein or fragments thereof (preferably antigen-binding fragments thereof) or immunoconjugates thereof. In some embodiments, the pharmaceutical composition further comprises pharmaceutic adjuvants.

The present invention also includes a composition (including a pharmaceutical composition or pharmaceutical formulation) comprising an antibody or an immunoconjugate thereof of the present invention and/or a composition (including a pharmaceutical composition or pharmaceutical formulation) comprising a polynucleotide encoding an antibody of the present invention. In certain embodiments, the composition comprises one or more antibodies or fragments thereof of the present invention or one or more polynucleotides encoding one or more antibodies or fragments thereof of the present invention.

These compositions may also contain suitable pharmaceutically acceptable adjuvants, such as pharmaceutically acceptable carriers, pharmaceutically acceptable excipients, including buffers or diluents known in the art.

As used herein, "pharmaceutically acceptable carrier" includes any and all physiologically compatible solvents, dispersion media, isotonic and absorption delaying agents, etc. Pharmaceutical carriers suitable for present invention can be sterile liquids, such as water and oils, including those derived from petroleum, animal, vegetable or synthesis, such as peanut oil, soybean oil, mineral oil, sesame oil, etc. Water is a preferred carrier when the pharmaceutical composition is administered intravenously. Saline solutions and aqueous dextrose and glycerol solutions can also be employed as liquid carriers, particularly for injectable solutions.

Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. See also "Handbook of Pharmaceutical Excipients"(fifth Edition, R. C. Rowe, P. J. Seskey and S. C. Owen, pharmaceutical Press, London, Chicago) for the use of excipients and uses thereof.

The compositions, if desired, can also contain minor amounts of humectants or emulsifiers, or pH buffers.

The compositions of the present invention can be in a variety of forms. Such forms include, for example, liquid, semi-solid and solid dosage forms, such as liquid solutions (e.g., injectable and infusible solutions), dispersions or suspensions, liposomes and suppositories. The preferred form depends on the intended mode of administration and therapeutic use. Commonly preferred compositions are in the form of injectable or infusible solutions. Preferred modes of administration are parenteral (e.g., intravenous, subcutaneous, intraperitoneal (i.p.), intramuscular) injection. In a preferred embodiment, the antibody molecule is administered by intravenous infusion or injection. In another preferred embodiment, the antibody molecule is administered by intramuscular, intraperitoneal, or subcutaneous injection.

Antibodies of the present invention binding to human cytomegalovirus may be lyophilized for storage and reconstituted in a suitable vehicle prior to use. This technique has been shown to be effective for conventional protein preparation, and well known lyophilization and reconstitution techniques can be employed.

In some embodiments, the present invention also provides kits comprising an antibody, a pharmaceutical composition, or an immunoconjugate of the present invention, and optionally a package insert for directing administration.

In some embodiments, the present invention also provides a pharmaceutical product comprising an antibody, a pharmaceutical composition, an immunoconjugate of the present invention, optionally further comprising a package insert for directing administration.

In some embodiments, the present invention also provides methods for preventing or treating a human cytomegalovirus infection or a human cytomegalovirus-related disease (HCMV-related disease) comprising administering an effective amount of an antibody or antigen-binding fragment thereof or immunoconjugate thereof or pharmaceutical composition of the present invention.

"HCMV-associated disease" refers to any disease associated with HCMV infection, including diseases that HCMV has been shown or can be considered to be the pathophysiological cause of the disease, or one of the causes that exacerbate the disease. For example, diseases caused by HCMV are also encompassed within the scope of HCMV-related diseases. In some embodiments, HCMV-associated diseases include, but are not limited to, various diseases such as (a) interstitial pneumonia, retinitis, gastroenteritis, encephalitis etc. resulting from reactivation of HCMV in immunodeficient states such as AIDS, cancer, after organ transplantation, bone marrow transplantation, hemodialysis, etc.; (b) Congenital HCMV infection caused by the spread of HCMV infection from the pregnant woman to the fetus; (c) abortion, stillbirth, and neonatal death caused by above-mentioned congenital HCMV infection; (d) low birth weight, hepatosplenomegaly, jaundice, thrombocytopenic purpura, microcephaly, mental developmental disorder, mental retardation, chorioretinitis or hearing impairment due to the above-mentioned congenital HCMV infection without death; (e) prophylactic or therapeutic agent for hepatic dysfunction, interstitial pneumonia or mononucleosis caused by HCMV infection in newborn or infant. Exemplary diseases are also described in Taya Keiko, Discussion of Infection: Cytomegalovirus infection, Infectious Diseases Weekly Report Japan.2003; Week 15, p10-14; Griffiths, P.D., The treatment of Cytomegalovirus infection. J. Anti. Chemo., 2002: 49: p243-253; Demmler, G.J., Congential cytomegalovirus infection and disease. Seminars in Pediatric Infection Diseases. Seminors in Pediatric Infectious Diseases, 1999; 10: p195-200.

In some embodiments, the present invention relates to a method of increasing, enhancing or stimulating resistance in a human subject infected with human cytomegalovirus, comprising administering an effective amount of an antibody or antigen-binding fragment thereof or immunoconjugate thereof or pharmaceutical composition of the present invention.

In some embodiments, individuals suitable for the methods of the present invention are those infected with HCMV.

In some embodiments, individuals suitable for the methods of the present invention are transplant patients, pregnant women, neonates, HIV-infected patients, cancer patients, or patients with autoimmune diseases.

It will be appreciated that any treatment can be performed using the immunoconjugates or compositions or kits of the present invention in place of or in addition to the antibodies of the present invention.

The mode of administration of the antibodies of the present invention (as well as pharmaceutical compositions or immunoconjugates comprising the same) can be any suitable route, such as parenteral administration, e.g., intradermal, intramuscular, intraperitoneal, intravenous or subcutaneous, mucosal (buccal, intranasal, intravaginal, rectal) or other means as will be appreciated by those skilled in the art. In some embodiments, the anti-human cytomegalovirus antibodies of the present invention can be administered to a patient by any suitable route, for example parenterally by intravenous (i.v.) infusion or bolus injection, intramuscularly or subcutaneously or intraperitoneally.

Various dosing schedules are encompassed herein, including, but not limited to, single dosing or multiple dosing at multiple time points, bolus dosing, and pulse infusion.

For the prevention or treatment of disease, the appropriate dosage of an antibody of the present invention will depend on the type of disease to be treated, the type of antibody, the severity and course of the disease, whether the antibody is administered for prophylactic or therapeutic purposes, previous therapy, the patient's clinical history and response to the antibody, and the discretion of the attending physician. The antibody is suitably administered to the patient in a single treatment or over a series of treatments. Typically, the clinician administers the composition until a dose is reached that achieves the desired effect. The antibodies of the present invention can thus be administered in a single dose or in two or more doses (which may contain the same or different amounts of the desired molecule) over a period of time, or by continuous infusion through an implanted device or catheter. Appropriate doses can be determined by using appropriate dose response data. In certain embodiments, antibodies can be administered to a patient over an extended period of time.

In certain embodiments, the anti-human cytomegalovirus antibodies or antigen-binding fragments thereof provided herein can be used to detect the presence of HCMV in a biological sample or to diagnose HCMV infection. The term "detection" as used herein includes quantitative or qualitative detection, exemplary detection methods may involve immunohistochemistry, immunocytochemistry, flow cytometry (e.g., FACS), antibody-coupled magnetic beads, ELISA assays, PCR-technology (e.g., RT-PCR). In certain embodiments, the biological sample is blood, serum, or other liquid sample of biological origin. In certain embodiments, the biological sample can be a tissue sample taken from, for example, salivary gland, lung, liver, pancreas, kidney, ear, eye, placenta, digestive tract, heart, ovary, pituitary, adrenal gland, thyroid, brain, or skin.

Methods of diagnosis can include contacting an antibody or antibody fragment with a sample. Methods of diagnosis may also include detection of antigen/antibody complexes.

In some embodiments, the method comprises contacting a biological sample with an anti-human cytomegalovirus antibody or antigen-binding fragment thereof as described herein under conditions that permit the binding of the anti-human cytomegalovirus antibody to HCMV, and detecting whether a complex is formed between the anti-human cytomegalovirus antibody and HCMV. The formation of complex indicates the presence of HCMV. The method may be an in vitro or in vivo method. In one embodiment, anti-human cytomegalovirus antibodies are used to select subjects suitable for treatment with anti-human cytomegalovirus antibodies, e.g., wherein HCMV is a biomarker used to select the subject.

In one embodiment, the antibody of the present invention can be used to diagnose the diseases described herein, e.g., to evaluate (e.g., monitor) the treatment or progression, diagnosis and/or staging of the diseases described herein in an individual. In certain embodiments, an anti-human cytomegalovirus antibody conjugated to a label is provided.

In some embodiments of any of the present inventions provided herein, the sample is obtained prior to treatment with an anti-human cytomegalovirus antibody. In some embodiments, the sample is obtained prior to treatment with the pharmaceutical composition described herein. In some embodiments, the sample is formalin-fixed or paraffin-embedded (FFPE). In some embodiments, the sample is a biopsy (e.g., a core biopsy), a surgical specimen (e.g., a specimen from surgical resection), or a fine needle aspirate.

In some embodiments, HCMV is detected prior to treatment, e.g., prior to initiation of treatment or prior to a certain treatment after a treatment interval.

In some embodiments, detection kits comprising the antibody or antigen-binding fragment thereof of the present invention are provided for diagnosing the diseases described herein, e.g., a HCMV-related disease or a HCMV infection.

In some embodiments, provided is a method of treating the diseases described herein, comprising: testing the presence of HCMV in a subject (e.g., sample) (e.g., a subject sample), thereby determining a HCMV value, comparing the HCMV value to a control value, and administering a therapeutically effective amount of an anti-human cytomegalovirus antibody (e.g., an anti-human cytomegalovirus antibody described herein) or an antigen-binding fragment thereof to the subject if the HCMV value is greater than the control value, thereby treating the diseases described herein. In some embodiments, the antibodies of the present invention can be used for passive immunization.

In some embodiments, the antibodies or fragments thereof of the present invention may also be used in kits for monitoring the production of vaccines with desired immunogenicity.

In some embodiments, the present invention also relates to a method of neutralizing human cytomegalovirus in an individual or sample, comprising contacting an antibody or antigen-binding fragment thereof of the present invention with the individual or sample and testing the capacity of the antibody or antigen-binding fragment thereof to bind to neutralize human cytomegalovirus. The present invention therefore also relates to the use of an antibody or antigen-binding fragment thereof of the present invention for the above-described method, as well as to the use of an antibody or antigen-binding fragment thereof of the present invention for the preparation of a medicament or a composition or a kit for the above-described method, and/or the use of an antibody or antigen-binding fragment thereof of the present invention in the preparation of a kit for the diagnosis of a disease as described herein.

The methods and uses applicable to the antibodies or antigen-binding fragments thereof of the present invention are equally applicable to immunoconjugates, compositions or kits comprising the antibodies or antigen-binding fragments thereof of the present invention.

The following describes the present invention in more detail with reference to accompanying drawings. However, these drawings and the specific embodiments of the present invention should not be considered as limiting the scope of the present invention, and modifications readily conceivable to those skilled in the art will be included within the spirit of the present invention and the scope of the appended claims.

### Brief Description of the Drawings

FIG.1 shows the results of neutralization of the HCMV standard strains Towne and TB40E in HFF cells with TRN1017, TRN1018, TRN1019, and TRN1020.
FIG.1A shows the results of neutralization of the HCMV standard strain Towne in HFF cells with four antibodies TRN1017, TRN1018, TRN1019, and TRN1020. FIG.1B shows the results of neutralization of HCMV strain TB40E infected in HFF cells with four antibodies TRN1017, TRN1018, TRN1019, and TRN1020.
FIG. 2 shows the binding affinity of antibodies TRN1017, TRN1018, TRN1019, and TRN1020, respectively, to the antigen gB glycoprotein.
FIG. 3 shows the effects of single point mutations at the 12 glycosylation sites (N208, N281, N284, N302, N341, N383, N405, N409, N417, N447, N452, and N456) of the gB glycoprotein on the binding activity of the antibodies of the present invention.
FIG. 4 shows linear structure of the extracellular segment of the gB glycoprotein and fusion domain thereof (including three mutation positions).

### Definitions

It is to be understood that the terms used herein are for the purpose of describing particular embodiments only, and are not intended to limit the scope of the present invention which will be limited only by the appended claims. Unless otherwise specified, all technical and scientific terms used herein have the same meanings as those generally understood by a person of ordinary skill in the art to which the present invention belongs.

For purposes of interpreting this specification, the following definitions will be used and whenever appropriate, terms used in the singular may also include the plural and vice versa. It is to be understood that the terms used herein are for the purpose of describing particular embodiments only, and are not intended to be limiting.

The term "about" when used in conjunction with a numerical value is intended to encompass numerical values within a range having a lower limit that is 5% less than the numerical value specified, and an upper limit that is 5% greater than the numerical value specified.

As used herein, the term "and/or" means any one of the alternatives or two or more of the alternatives.

As used herein, the terms "comprising" or "including" means including the recited elements, integers or steps but not excluding any other elements, integers or steps. As used herein, the terms "comprising" or "including" when used herein, unless otherwise indicated, encompass the stated elements, integers, or steps. For example, reference to an antibody variable region "comprising" a particular sequence is also intended to encompass antibody variable regions consisting of that particular sequence.

"Human Cytomegalovirus" (HCMV) is a DNA double-helix virus of the genus Cytomegalovirus in the subfamily of Herpesviruses, also known as human herpesvirus type 5 (HHV-5). As used herein, "human cytomegalovirus", "HCMV", "human herpes virus type 5", "HHV-5" are all generic.

The "gB glycoprotein of human cytomegalovirus (HCMV)" (or "HCMV gB glycoprotein" or "HCMB-gB glycoprotein") is one of the major glycoproteins constituting the outer shell of HCMV, which is known to contribute to the invasion of viral particles into cells, cell fusion, and intercellular infection of viruses. The amino acid sequence of the HCMV gB glycoprotein can be obtained from the publicly available sequence database NCBI GENE.

"Fusion domain of the gB glycoprotein of human cytomegalovirus (HCMV)" or "HCMV gB glycoprotein fusion domain" refers to a region consisting of contiguous amino acid residues from position 150-250 in the amino acid sequence of the HCMV gB glycoprotein (Heidi G. Burke et al. Crystal Structure of the Human Cytomegalovirus Glycoprotein B. 2015).

A "complementarity determining region" or "CDR region" or "CDR" is a region of an antibody variable domain that is highly variable in sequence and forms a structurally defined loop ("hypervariable loop") and/or includes an antigen-contacting residue ("antigen-contacting point"). The CDRs are primarily responsible for the binding to antigenic epitopes. The CDRs of the heavy and light chains are commonly referred to as CDR1, CDR2 and CDR3, numbered sequentially from the N-terminus. CDRs located within the variable domain of the heavy chain of an antibody are referred to as HCDR1, HCDR2, and HCDR3, while CDRs located within the variable domain of the light chain of an antibody are referred to as LCDR1, LCDR2, and LCDR3. Within a given light chain variable region or heavy chain variable region amino acid sequence, the precise amino acid sequence boundary for each CDR can be determined using any one or combination of a number of well-known antibody CDR assignment systems, including, for example: Chothia based on the three-dimensional structure of antibodies and the topology of the CDR loops (Chothia et al. (1989) Nature 342: 877-883, Al-Lazikani et al. "Standard conformations for the canonical structures of immunoglobulins", Journal of Molecular Biology, 273, 927-948 (1997)), Kabat based on antibody sequence variability (Kabat et al. Sequences of Proteins of Immunological Interest, 4th Ed. U.S. Department of Health and Human Services, National Institutes of Health (1987)), AbM (University of Bath), Contact (University College London), International ImMunoGeneTics database(IMGT) (On the World Wide Web at imgt.cines.fr/), and North CDR definitions based on affinity propagation clustering using a large number of crystal structures.

For example, according to different CDR determination schemes, the residues of each CDR are defined as follows.

| CDR | Kabat Scheme | AbM Scheme | Chothia Scheme | Contact Scheme |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 | H31-H35B | H26-H35B | H26-H32 | H30-H35B |
| (Kabat Numbering system) | | | | |
| HCDR1 | H31-H35 | H26-H35 | H26-H32 | H30-H35 |
| (Chothia Numbering system) | | | | |
| HCDR2 | H50-H65 | H50-H58 | H53-H55 | H47-H58 |
| HCDR3 | H95-H102 | H95-H102 | H96-H101 | H93-H101 |
| (Kabat Numbering system) | | | | |

| | | | | |
|---|---|---|---|---|
| CDRs can also be determined based on the same Kabat numbering positions as the reference CDR sequences (any of the exemplary CDRs in the present invention). | | | | |

Unless otherwise indicated, in the present invention, the term "CDR" or "CDR sequence" encompasses CDR sequences determined in any of the ways described above.

Unless otherwise stated, in the present invention, reference to residue positions in an antibody variable region, including heavy chain variable region residues and light chain variable region residues, refers to numbering positions according to the Kabat numbering system (Kabat et al. Sequences of Proteins of Immunological Interest, 5th Ed. Public Health Service, National Institutes of Health, Bethesda, Md. (1991)).

In one embodiment, the CDR boundary of the antibody of the present invention is determined by IMGT rules, for example using the IMGT database.

It should be noted that the boundaries of the CDRs of variable regions of the same antibody obtained based on different assignment systems may differ. That is, the CDR sequences of the variable regions of the same antibody defined under different assignment systems are different. Thus, in reference to defining an antibody with a specific CDR sequence as defined herein, the scope of the antibody also encompasses antibodies whose variable region sequences comprise the specific CDR sequence, but whose claimed CDR boundaries differ from the specific CDR boundaries as defined herein due to the application of different schemes (e.g., different assignment system rules or combinations).

As used herein, the term "neutralization" refers to the ability of neutralizing a pathogen to initiate and/or maintain infection in a host.

As used herein, the term "epitope" refers to a portion of an antigen (e.g., the gB glycoprotein of HCMV) that specifically interacts with an antibody molecule. Epitopes within a protein antigen can be formed from contiguous amino acids (usually linear epitopes) or discrete amino acids (usually conformational epitopes) juxtaposed by the tertiary folding of the protein. Epitopes formed from contiguous amino acids are typically (but not always) remain exposed to denaturing solvents, while epitopes formed by tertiary folding are typically lost upon treatment with denaturing solvents.

"An antibody that binds to the same or an overlapping epitope" as a reference antibody refers to an antibody that blocks 50%, 60%, 70%, 80%, 90%, or 95% or more of the binding of the reference antibody to its antigen in a competition assay, and conversely, a reference antibody blocks 50%, 60%, 70%, 80%, 90% or 95% or more of the binding of the antibody to its antigen in a competition assay.

An antibody that competes with a reference antibody for binding to its antigen refers to an antibody that blocks 50%, 60%, 70%, 80%, 90%, or 95% or more of the binding of the reference antibody to its antigen in a competition assay. Conversely, a reference antibody blocks 50%, 60%, 70%, 80%, 90%, or 95% or more of the binding of the antibody to its antigen in a competition assay. Numerous types of competition assays can be used to determine whether one antibody competes with another, such as ELISA, SPR, solid phase direct or indirect radioimmunoassay (RIA), solid phase direct or indirect enzyme immunoassay (EIA) (see, e.g., Stahli et al. 1983, Methods in Enzymology 9:242-253)).

An antibody that inhibits (e.g., competitively inhibits) the binding of a reference antibody to its antigen refers to an antibody that inhibits 50%, 60%, 70%, 80%, 90%, or 95% or more of the binding of the reference antibody to its antigen. Conversely, a reference antibody inhibits 50%, 60%, 70%, 80%, 90% or 95% or more of the binding of the antibody to its antigen. The binding of an antibody to its antigen can be measured by affinity (e.g., equilibrium dissociation constant). Methods for determining affinity are known in the art.

An antibody that exhibits the same or similar binding affinity and/or specificity as a reference antibody refers to an antibody that is capable of having a binding affinity and/or specificity of at least 50%, 60%, 70%, 80%, 90% or 95% or more of that of the reference antibody. This can be determined by any method known in the art for determining binding affinity and/or specificity.

"An antibody in the form of IgG" refers to the IgG form to which the heavy chain constant region of an antibody belongs. The heavy chain constant regions are the same for all antibodies of the same type, and differ between antibodies of different types. For example, an antibody in the form of IgG1 refers to a IgG3 domain whose heavy chain constant region Ig domain is IgG1.

A "human" antibody (HuMAb) refers to an antibody having a variable region in which both the framework and CDR regions are derived from human germline immunoglobulin sequences. In addition, if the antibody contains constant regions, the constant regions are also derived from human germline immunoglobulin sequences.

A "humanized" antibody refers to an antibody in which some, most, or all of the amino acids outside the CDR domain of a non-human antibody (e.g., a mouse antibody) have been replaced with the corresponding amino acids from a human immunoglobulin. In one embodiment of the humanized form of the antibody, some, most or all of the amino acids outside of the CDR domain have been replaced with amino acids from a human immunoglobulin, while some, most or all of the amino acids within one or more of the CDR regions have not been altered. Small additions, deletions, insertions, substitutions or modifications of amino acids are permissible so long as they do not eliminate the ability of the antibody to bind a particular antigen. A "humanized" antibody retains similar antigen specificity as the original antibody.

As used herein, a "chimeric antibody" refers to an antibody in which the variable region is derived from one species and the constant region is derived from another species, such as an antibody in which the variable region is derived from a mouse antibody and the constant region is derived from a human antibody.

As used herein, an "antibody fragment" refers to a molecule that differs from an intact antibody, which comprises a portion of the intact antibody and binds to the antigen to which the intact antibody binds. As used herein, the term "antigen-binding fragment" as used herein refers to one or more fragments of an antibody that retain the ability to specifically bind to an antigen (e.g., the gB glycoprotein of human HCMV). Examples of antibody fragments include, but are not limited to, Fv, Fab, Fab', Fab'-SH, F(ab')₂, diabodies, linear antibodies, single chain antibodies (e.g., scFv), single domain antibodies, bivalent antibodies or bispecific antibodies or fragments thereof, camelid antibodies, and bispecific or multispecific antibodies formed from antibody fragments.

As used herein, "multispecific" refers to an antibody that specifically binds to at least two different antigens or two different epitopes within an antigen, e.g., three, four, or five different antigens or epitopes.

As used herein, "bispecific" refers to an antibody that specifically binds to two different antigens or two different epitopes within the same antigen. Bispecific antibodies may be cross-reactive with other related antigens, or can bind to epitopes shared between two or more different antigens.

An "immunoconjugate" is an antibody conjugated to one or more other substances, including but not limited to labels.

As used herein, the term "label" refers to a compound or composition that is directly or indirectly conjugated or fused to an agent, such as a polynucleotide probe or antibody, and facilitates the detection of the agent to which it is conjugated or fused. The label may be detectable by itself (e.g., a radioisotope label or a fluorescent label) or, in the case of an enzymatic label, may catalyze chemical alteration of the detectable substrate compound or composition. The term is intended to encompass direct labeling of the probe or antibody by coupling (i.e., physically linking) a detectable substance to the probe or antibody, as well as indirect labeling of the probe or antibody by reaction with another reagent that is directly labeled. Examples of indirect labeling include the detection of a primary antibody using a fluorescently labeled secondary antibody and end-labeling of a DNA probe with biotin such that it can be detected with fluorescently labeled streptavidin.

A "vector" refers to a polynucleotide that is capable of replicating within a biological system or is moveable between such systems. Vector polynucleotides typically contain elements such as an origin of replication, a polyadenylation signal, or a selectable marker of which the function is to facilitate the replication or maintenance of the polynucleotide in a biological system. Examples of such biological systems may include cells, viruses, animals, plants, and biological systems reconstituted with biological components capable of replicating vectors. The polynucleotide comprising the vector may be a DNA or RNA molecule or a hybrid molecule of these molecules. An "expression vector" refers to a vector that can be used in a biological system or a reconstituted biological system to direct translation of a polypeptide encoded by a polynucleotide sequence present in the expression vector.

An "isolated" antibody is an antibody that has been separated from components of its natural environment. In some embodiments, the antibody is purified to greater than 95% or 99% purity as determined, for example, by electrophoresis (e.g., SDS-PAGE, IEF, capillary electrophoresis) or chromatography (e.g., ion exchange or reverse phase HPLC). For a review of methods for assessing antibody purity, see, e.g., Flatman et al. J. Chromatogr. B848: 79-87 (2007)).

An "isolated" nucleic acid refers to a nucleic acid molecule that has been separated from components of its natural environment. An isolated nucleic acid includes a nucleic acid molecule contained in a cell that normally contains the nucleic acid molecule, but which is present extrachromosomally or at a chromosomal location other than its natural chromosomal location.

Calculation of sequence identity between sequences was performed as follows.

To determine the percent identity of two amino acid sequences or two nucleic acid sequences, the sequences are aligned for optimal comparison purposes (e.g., gaps may be introduced in one or both of the first and second amino acid sequences or nucleic acid sequences for optimal alignment or non-homologous sequences may be discarded for comparison purposes). In a preferred embodiment, for comparison purposes, the length of the aligned reference sequence is at least 30%, preferably at least 40%, more preferably at least 50%, 60% and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position.

Sequence comparison between two sequences and calculation of percent identity can be accomplished using mathematical algorithms. In a preferred embodiment, the percent identity between two amino acid sequences was determined using the Needlema and Wunsch ((1970) J. Mol. Biol48:444-453) algorithms (available at http:// www.gcg.com) that have been integrated into the GAP program of the GCG software package, using either the Blossum 62 matrix or the PAM250 matrix and the GAP weights 16, 14, 12, 10, 8, 6 or 4 and the length weights 1, 2, 3, 4, 5 or 6. In yet another preferred embodiment, the percent identity between two amino acid sequences was determined using the GAP program in the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred set of parameters (and one set of parameters that should be used unless otherwise stated) is the Blossum 62 scoring matrix with a gap penalty of 12, a gap extension penalty of 4 and a frameshift gap penalty of 5.

The percent identity between two amino acid or nucleotide sequences can also be determined using the algorithm of E. Meyers and W. Miller ((1989) CABIOS, 4:11-17) which has been incorporated into the ALIGN program (version 2.0), using the PAM120 weighted remainder table, a gap length penalty of 12 and a gap penalty of 4.

Additionally or alternatively, the nucleic acid sequences and protein sequences described herein can be further used as "query sequences" to perform searches against public databases, e.g., to identify other family member sequences or related sequences.

As used herein, the term "hybridizes under low stringency, medium stringency, high stringency, or very high stringency conditions" describes the hybridization and wash conditions. Guidance for performing hybridization reactions can be found in Current Protocols in Molecular Biology, John Wiley & Sons, N.Y.(1989), 6.3.1-6.3.6, incorporated by reference. Both aqueous and non-aqueous methods are described in the references and either method can be used. Specific hybridization conditions referred to herein are as follows: 1) the low stringency hybridization condition including washing twice in 6X sodium chloride/sodium citrate (SSC) at about 45°C, followed by in 0.2X SSC, 0.1% SDS at least 50°C(for low stringency conditions, the temperature for washing can be increased to 55°C); 2)the medium stringency hybridization condition including washing once or more times in 6X SSC at about 45°C, followed by in 0.2X SSC, 0.1% SDS at 60°C; 3)the high stringency hybridization condition including washing once or more times in 6X SSC at about 45°C, followed by in 0.2X SSC, 0.1% SDS at 65°C; and preferably 4) the very high stringency hybridization condition including washing once or more times in 0.5 M sodium phosphate, 7% SDS at 65°C,followed by 0.2X SSC, 0.1% SDS at 65°C. The very high stringency condition (4) is the preferred condition and the one that should be used unless otherwise stated. The terms "host cell", "host cell line" and "host cell culture" are used interchangeably and refer to a cell into which an exogenous nucleic acid is introduced, including progeny of such a cell. Host cells include "transformants" and "transformed cells", which include primarily transformed cells and progeny derived therefrom, regardless of the number of passages. The progeny may not be exactly the same as the parent cell in nucleic acid content, but may contain mutations. Mutant progeny having the same function or biological activity as screened or selected in the initially transformed cell are included herein.

The term "pharmaceutical auxiliary material" refers to a diluent, adjuvant (e.g., Freund's adjuvant (complete and incomplete)), excipient, carrier or stabilizer, etc., with which the active substance is administered.

The term "pharmaceutical composition" refers to a composition that is present in a form that allows the biological activity of the active ingredients contained therein to be effective, and which does not contain additional ingredients having unacceptable toxicity to the subject to which the composition is administered.

The term "effective amount" refers to an amount or dose of an antibody or fragment or conjugate or composition of the present invention which, upon administration to a patient in a single or multiple dose, produces the desired effect in the patient in need of treatment or prevention. An effective amount can be readily determined by the attending physician, as one skilled in the art, by considering the following factors: such as species of mammals: size, age and general health; specific diseases involved; the extent or severity of the disease; response of an individual patient; the specific antibody administered; modes of administration; bioavailability characteristics of the administered formulation; a selected dosing regimen; and the use of any concomitant therapy.

A "therapeutically effective amount" refers to an amount effective, at dosages and for periods of time required, to achieve the desired therapeutic result. The therapeutically effective amount of the antibody or antibody fragment or conjugate or composition thereof can vary depending on factors such as the disease state, the age, sex, and weight of the subject, and the ability of the antibody or antibody portion to elicit a desired response in the subject. A therapeutically effective amount is also one in which any toxic or adverse effect of the antibody or antibody fragment or conjugate or composition thereof is less than a therapeutically beneficial effect. A "therapeutically effective amount" preferably inhibits a measurable parameter by at least about 20%, more preferably by at least about 40%, even more preferably by at least about 50%, 60%, or 70% and still more preferably by at least about 80% or 90% relative to an untreated individual. The ability of a compound to inhibit a measurable parameter can be evaluated in an animal model system predictive of efficacy in human autoimmune disease or inflammation.

A "prophylactically effective amount" refers to an amount effective, at dosages and for periods of time required, to achieve the desired prophylactic result. Generally, a prophylactically effective amount will be less than a therapeutically effective amount because a prophylactic dose is used in an individual prior to or at an earlier stage of the disease.

As used herein, "individual" or "subject" is used interchangeably and includes mammals, such as human.

As used herein, "treating" refers to slowing, interrupting, arresting, relieving, stopping, reducing, or reversing the progression or severity of an existing symptom, disorder, condition, or disease.

As used herein, "preventing" includes the inhibition of the occurrence or development of a disease or disorder or the symptoms of a particular disease or disorder. In some embodiments, a subject or pregnant woman with a family history of an immune system disease (autoimmune disease or immune compromised) is a candidate for a prophylactic regimen. In general, in the context of an immune system disease (autoimmune disease or Immunocompromisation), the term "prevention" refers to the administration of a drug prior to the onset of a sign or symptom of an immune system disease (autoimmune disease or Immunocompromisation), particularly in a subject at risk for an immune system disease (autoimmune disease or Immunocompromisation).

A "subject/patient sample" refers to a collection of cells or fluids obtained from a patient or subject. The source of the tissue or cell sample may be a solid tissue, such as fresh, frozen and/or preserved organs or tissue samples or biopsy samples or puncture samples; blood or any blood component; body fluids such as cerebrospinal fluid, amniotic fluid, peritoneal fluid (ascites fluid), or interstitial fluid; cells from a subject at any time of gestation or development. Tissue samples may contain compounds that are not naturally intermixed with tissue in nature, such as preservatives, anticoagulants, buffers, fixatives, nutrients, antibiotics, etc.

These and other aspects and embodiments of the present invention are described in the accompanying drawings and the following detailed description of the present invention and are exemplified in the following examples. Any or all of the features discussed above and throughout this application may be combined in various embodiments of the present invention. The following examples further illustrate the present invention, however, it should be understood that the examples have been described by way of illustration and not limitation, and that various modifications may be made by those skilled in the art.

### Examples

### Example 1 Design of sequences of the specific antigen HCMV glycoprotein gB

Information on sequences of glycoprotein gB of 56 human cytomegalovirus strains (including experimental and clinical strains) was retrieved from NCBI GENG database, and the homology of gB glycoprotein was more than 70% through amino acid sequence alignment. The gB glycoprotein of standard strain Towne was selected as one specific antigen (gB_Towne strain), with the sequence shown in SEQ ID NO: 19, while the homologous sequence of gB glycoprotein of 56 virus strains was selected as another specific antigen (gB_Con. Strain), with the sequence shown in SEQ ID NO: 20, for screening a broad-spectrum monoclonal antibody against human cytomegalovirus.
SEQ ID NO: 19 
MESRIWCLVVCVNLCIVCLGAAVSSSSTRGTSATHSHHSSHTTSAAHSRSGSVSQRVTSSQT
VSHGVNETIYNTTLKYGDVVGVNTTKYPYRVCSMAQGTDLIRFERNIVCTSMKPINEDLD
EGIMVVYKRNIVAHTFKVRVYQKVLTFRRSYAYIHTTYLLGSNTEYVAPPMWEIHHINSHS
QCYSSYSRVIAGTVFVAYHRDSYENKTMQLMPDDYSNTHSTRYVTVKDQWHSRGSTWLY
RETCNLNCMVTITTARSKYPYHFFATSTGDVVDISPFYNGTNRNASYFGENADKFFIFPNY
TIVSDFGRPNSALETHRLVAFLERADSVISWDIQDEKNVTCQLTFWEASERTIRSEAEDSYH
FSSAKMTATFLSKKQEVNMSDSALDCVRDEAINKLQQIFNTSYNQTYEKYGNVSVFETTG
GLVVFWQGIKQKSLVELERLANRSSLNLTHNETKESTDGNNATHLSNMESVHNLVYAQLQ
FTYDTLRGYINRALAQIAEAWCVDQRRTLEVFKELSKINPSAILSAIYNKPIAARFMGDVL
 
GLASCVTINQTSVKVLRDMNVKESPGRCYSRPVVIFNFANSSYVQYGQLGEDNEILLGNH
RTEECQLPSLKIFIAGNSAYEYVDYLFKRMIDLSSISTVDSMIALDIDPLENTDFRVLELYSQ
KELRSINVFDLEEIMREFNSYKQRVKYVEDKGLNDIFEAQKIEWHELEVLFQGPGHHHHH
HH
SEQ ID NO: 20

MESRIWCLVVCVNLCIVCLGAAVSSSSTSHATSSTHNGSHTSRTTSAQTRSVSQHVTSSEAV
SHRANETIYNTTLKYGDVVGVNTTKYPYRVCSMAQGTDLIRFERNIVCTPMKPINEDLDE
GIMVVYKRNIVAHTFKVRVYQKVLTFRRSYAYIHTTYLLGSNTEYVAPPMWEIHHINSHSQ
CYSSYSRVIAGTVFVAYHRDSYENKTMQLMPDDYSNTHSTRYVTVKDQWHSRGSTWLYR
ETCNLNCMVTITTARSKYPYHFFATSTGDVVDISPFYNGTNRNASYFGENADKFFIFPNYTI
VSDFGRPNSAPETHRLVAFLERADSVISWDIQDEKNVTCQLTFWEASERTIRSEAEDSYHFS
SAKMTATFLSKKQEVNMSDSALDCVRDEAINKLQQIFNTSYNQTYEKYGNVSVFETTGGL
VVFWQGIKQKSLVELERLANRSSLNLTHETKESTDGNNTTHLSNMESVHNLVYAQLQFTY
DTLRGYINRALAQIAEAWCVDQRRTLEVFKELSKINPSAILSAIYNKPIAARFMGDVLGLA
SCVTINQTSVKVLRDMNVKESPGRCYSRPVVIFNFANSSYVQYGQLGEDNEILLGNHRTEE
CQLPSLKIFIAGNSAYEYVDYLFKRMIDLSSISTVDSMIALDIDPLENTDFRVLELYSQKELR
SSNVFDLEEIMREFNSYKQRVKYVEDKGLNDIFEAQKIEWHELEVLFQGPGHHHHHHH.

### Example 2 Screening of HCMV gB glycoprotein serum antibody

### 1. Screening process

Immune-compromised volunteers were recruited from different hospitals, and 272 whole blood samples were obtained from eligible patients; human peripheral blood mononuclear cells (PBMC) and plasma were isolated with Ficoll lymphocyte separation medium (GE). The two designed HCMV gB glycoproteins were expressed and purified by conventional methods at a concentration of 2µg/mL for later use. 272 blood samples (separated plasma) were tested for antibody titer (serum antibody titer measured by EC50) by ELISA assay and cancer was classified.

### 2. Results

272 clinical blood samples belong to 17 different cancer disease types, and the result showed that 268 samples were seropositive, with a positive rate as high as 98.53%, which was consistent with the conclusion reported by the study on the prevalence of HCMV infection in human population.

These samples were classified into patients with glioblastoma, lung cancer, rectal cancer, liver cancer, gastric cancer, esophageal cancer, colon cancer, kidney cancer, ovarian cancer, breast cancer, pancreatic cancer, prostate cancer, bladder cancer, gallbladder cancer, cervical cancer, and lupus erythematosus, etc, respectively, indicating that HCMV widely exists in immune-compromised populations such as cancer patients and patients with autoimmune diseases. Anti-human cytomegalovirus antibodies exist in these patients, so anti-human cytomegalovirus monoclonal antibodies can be screened from their B cells.

### Example 3 Isolation of anti-human cytomegalovirus monoclonal antibody gene

### 1. Sorting of gB_Towne strain and gB_Con. Strain protein antigen-specific B cells

To improve the sorting yield of memory B cells, the sample numbered Es0018 in Example 3 was selected for specific sorting with two HCMV gB glycoproteins (gB_Towne strain and gB_Con. Strain) at the same time. The steps for screening are as follows:
Single memory B lymphocytes can be obtained from the gB antigen-specific memory B lymphocytes in human peripheral blood in a single cell's sorting mode by using flow cytometry (BD FACSAria) according to the manufacturer's instructions, applying the logic gates set up according to CD3-/ CD14-/ CD16-/ CD235a- / CD19+/ SIgD-/-labeled gB_Towne and gB_Con. +. To increase the specificity of the sorting, the labeled gB_Towne and gB_Con were both labeled with two-color fluorescence (PE-Cy7 and Bv421), resulting in double-positive cells on the diagonal and single-positive cells within the logic gate.

### 3. Amplification of single cell antibody variable region gene

The steps are as follows: A complete set of RT-PCR/PCR primers and experimental conditions have been successfully designed and established to amplify all known seven human-derived antibody heavy chain families and thirteen light chain families, including antibody variable region genes from IgG, IgA, IgM, IgD, and IgE (see CN107760690B).

### 4. Results

A plurality of memory B cells were isolated from a sample, and antibody gene sequences were obtained after amplification of antibody variable region genes in a single cell using the designed primers, with a somatic mutation rate of antibody heavy chain VH gene fragment ranging from 10% to 18.5%. After the gene amplification of the variable region of the single cell antibody, 103 gene sequences of the heavy chain variable region of the antibody and 109 gene sequences of the light chain variable region of the antibody were obtained respectively, after the pairing, the positive rate of the detected antibody was 32.4%, the somatic mutation rate of the heavy chain of the positive antibody was 7% to 16.5%, and the light chain was 2% and 15%.

### Example 4 Expression of anti-human cytomegalovirus monoclonal antibody

### 1. Construction of expression vectors

Linear expression vectors were constructed for the heavy chain variable region gene sequence and the light chain variable region gene sequence of the above-mentioned successfully paired antibody, respectively, and the isolated heavy chain and light chain variable region genes were respectively combined with their respective constant region sequences using the method of Overlapping PCR (Crystal Structure of the Human Cytomegalovirus Glycoprotein B, Heidi G. Burke and Ekaterina E. Heldwein, PLoS Pathog. 2015 Oct; 11(10): e1005227) into full length heavy and light chain genes, and recombinant antibodies were expressed for antibody screening and identification.

### 2. Screening positive antibodies

The above-mentioned expression vector (PCDNA 3.1) containing the heavy and light chain genes was transfected into HEK293 cells using a transfection reagent (Quigen) and cultured in a 12-well plate with DMEM medium containing FBS for 48 hours. After 48 hours, the cell culture supernatant was collected and tested for antibody expression and binding to the antigen HCMV gB glycoprotein (gB_Towne strain and gB_Con. Strain) by ELISA. The standard is TRN006 (CN 103910796 B), and the initial concentration is 0.5 µg/mL; the initial concentration of cell culture supernatant is the stock solution, 10 fold dilution in 4 series; mouse anti-human IgG Fab-HRP (Abcam) was diluted with commercially available SuperBlock in 1:10000. After the reaction is terminated, the dual-wavelength readings of 450 nm (sample) and 630 nm (microplate well) were detected with a multi-purpose microplate reader, and the final calculated values were OD450-OD630. The points with good linear relationship (R2 >0.99) between the value of TRN006 OD450-OD630 and the corresponding concentration were selected to establish the linear regression equation, and the antibody concentration was calculated with the sample value falling within the linear interval.

### 3. Identification results

Finally, the linear expression vectors were constructed for the heavy and light chains of 134 antibodies, respectively, which were successfully paired, and were co-transfected into HEK293 cells; 42 positive binding antibodies were screened, with a positive rate of 32.3%, wherein 36 antibodies showed strongly positive for gB_Towne strain, and 24 antibodies showed strongly positive for gB_Con. Strain and gB_Towne strain.

Of the four antibodies of the present invention screened: the heavy chain may have CDR1 of SEQ ID NO: 1-4, and/or CDR2 of SEQ ID NO: 5-8, and/or CDR3 of SEQ ID NO: 9-12. In particular examples, the light chain has CDR1 of SEQ ID NO: 21-24, and/or CDR2 of SEQ ID NO: 25-28, and/or CDR3 of SEQ ID NO: 29-32.

### TRN1017

24 antibodies showing strong positive for both gB_Con. Strain and gB_Towne strain were identified by DNA sequencing (Invitrogen) of the corresponding DNA preparations. One of the antibodies was identified as TRN1017 having:
a heavy chain variable region amino acid sequence as shown in SEQ ID NO: 13, and a light chain variable region amino acid sequence as shown in SEQ ID NO: 33;
a heavy chain amino acid sequence as shown in SEQ ID NO: 37, and a light chain amino acid sequence as shown in SEQ ID NO: 38.

### TRN1018

24 antibodies showing strong positive for both gB_Con. Strain and gB_Towne strain were identified by DNA sequencing of the corresponding DNA preparations. One of the antibodies was identified as TRN1018 having:
a heavy chain variable region amino acid sequence as shown in SEQ ID NO: 14, and a light chain variable region amino acid sequence as shown in SEQ ID NO: 34;
a heavy chain amino acid sequence as shown in SEQ ID NO: 39, and a light chain amino acid sequence as shown in SEQ ID NO: 40.

### TRN1019

24 antibodies showing strong positive for both gB_Con. Strain and gB_Towne strain were identified by DNA sequencing of the corresponding DNA preparations. One of the antibodies was identified as TRN1019 having:
a heavy chain variable region amino acid sequence as shown in SEQ ID NO: 15, and a light chain variable region amino acid sequence as shown in SEQ ID NO: 35;
a heavy chain amino acid sequence as shown in SEQ ID NO: 41, and a light chain amino acid sequence as shown in SEQ ID NO: 42.

### TRN1020

24 antibodies showing strong positive for both gB_Con. strain and gB_Towne strain were identified by DNA sequencing of the corresponding DNA preparations. One of the antibodies was identified as TRN1020 having:
a heavy chain variable region amino acid sequence as shown in SEQ ID NO: 16, and a light chain variable region amino acid sequence as shown in SEQ ID NO: 36;
a heavy chain amino acid sequence as shown in SEQ ID NO: 43, and a light chain amino acid sequence as shown in SEQ ID NO: 44.

The amino acid sequences of the above variable regions were analyzed, and the CDR sequences of these four antibodies were determined as follows using the IMGT numbering rule:

| Antibody | Heavy chain variable region | VH CDR1 | VH CDR2 | VH CDR3 |
|---|---|---|---|---|
| TRN1017 | SEQ ID NO: 13 | SEQ ID NO: 1 | SEQ ID NO: 5 | SEQ ID NO: 9 |
| TRN1018 | SEQ ID NO: 14 | SEQ ID NO: 2 | SEQ ID NO: 6 | SEQ ID NO: 10 |
| TRN1019 | SEQ ID NO: 15 | SEQ ID NO: 3 | SEQ ID NO: 7 | SEQ ID NO: 11 |
| TRN1020 | SEQ ID NO: 16 | SEQ ID NO: 4 | SEQ ID NO: 8 | SEQ ID NO: 12 |

| Antibody | Light chain variable region | VL CDR1 | VL CDR2 | VL CDR3 |
|---|---|---|---|---|
| TRN1017 | SEQ ID NO: 33 | SEQ ID NO: 21 | SEQ ID NO: 25 | SEQ ID NO: 29 |
| TRN1018 | SEQ ID NO: 34 | SEQ ID NO: 22 | SEQ ID NO: 26 | SEQ ID NO: 30 |
| TRN1019 | SEQ ID NO: 35 | SEQ ID NO: 23 | SEQ ID NO: 27 | SEQ ID NO: 31 |
| TRN1020 | SEQ ID NO: 36 | SEQ ID NO: 24 | SEQ ID NO: 28 | SEQ ID NO: 32 |

### Example 5 Evaluation of the antiviral neutralizing capacity of monoclonal antibodies against human cytomegalovirus

### 1. Evaluation of antiviral neutralizing capacity of antibodies in HFF cells

The cellular model HFF, as well as the standard strains Towne and TB40E of HCMV (both purchased from ATCC) were chosen for the evaluation of the antibody neutralizing capacity, the controls were the already reported antibodies SM5_1 and SM10, respectively (Crystal Structure of the Human Cytomegalovirus Glycoprotein B, Heidi G. Burke and Ekaterina E. Heldwein, PLoS Pathog. 2015 Oct:11(10): e1005227). Prior to infection, 5.00E+03 HFF cells per well were plated in a 96-well plate, with cell density being about 90%. The antibody with an initial concentration of 100 µg/mL was diluted 3-fold in 8 series in PBS, 10 µL dilution was added to each well, and 10 µL DMEM medium was added to virus control group. The cells were infected with a virus dose of MOI = 0.96, i.e. 40 µL/well of virus stock solution was added, the mixture was incubated in a 37°C incubator for 1 hour as virus infection solution. The cell supernatant was discarded from the 96-well plate, followed by rinsing twice with 100 µL PBS. 50 µL of virus infection solutions with different antibody dilutions were added to the 96-well plate and the mixture was infected for 4 hours in a 37°C incubator. The virus infection solution was aspirated and rinsed once with 100 µL PBS. 100 µL DMEM medium was added to each well, and cultured in an incubator containing 5% CO₂ at 37°C for 96 hours, during which the cell status was observed and recorded every day. The cell culture supernatant was discarded and rinsed twice with PBS. 50 µL of 4% paraformaldehyde was added to each well; the plate was placed at room temperature for 30 minutes to fix the cells, and rinsed twice with PBS. Cell nucleus were stained 15 minutes with 100 µL of 10 µg/mL Hoechst and rinsed 3 times with 200 µL of PBS (the 96-well plate was gently shaken), the nuclei staining was observed with blue light and viral infection was observed with green light. The fluorescence intensity of the emitted light at 535 nm was measured under excitation light at 485 nm, and the relative fluorescence intensity was used to describe the degree of viral infection of cells and the effect of antibodies against viral infection. Calculation results (Formula: anti-viral infection effect = 100 - fluorescence intensity of antibody virus complex group/fluorescence intensity of virus group × 100)

### 2. Evaluation of antiviral neutralizing capacity of antibodies in primary HUVEC cells

Antibodies were evaluated for their capacity to resist Towne infection in primary HUVEC cell lines. Prior to infection, 4.00E+04 HUVEC cells per well were plated in a 96-well plate, with cell density being about 80%. The antibody with an initial concentration of 100 µg/mL was diluted 3-fold in 8 series, 10 µL dilution was added to each well, and 10 µL medium was added to virus control group. The cells were infected with a virus dose of MOI= 5, i.e. 40 µL/well of virus stock solution was added, the mixture was incubated in a 37°C incubator for 1 hour as virus infection solution. The cell supernatant was discarded from the 96-well plate, followed by rinsing twice with 100 µL PBS. 50 µL of virus infection solutions with different antibody dilutions were added to the 96-well plate and the mixture was infected for 4 hours in a 37°C incubator. The virus infection solution was aspirated and supplemented with 100 µL DMEM medium per well, the mixture was cultured in an incubator containing 5% CO₂ at 37°C for 7 days, during which the cell status was observed and recorded every day. The cell culture supernatant was discarded and rinsed twice with PBS. 50 µL of 4% paraformaldehyde was added to each well; the plate was placed at room temperature for 30 minutes to fix the cells, and rinsed twice with PBS. Cell nucleus were stained 15 minutes with 100 µL of 10 µg/mL Hoechst and rinsed 3 times with 200 µL of PBS (the 96-well plate was gently shaken), the nuclei staining was observed with blue light and viral infection was observed with green light. The fluorescence intensity of the emitted light at 535 nm was measured under excitation light at 485 nm, and the relative fluorescence intensity was used to describe the degree of viral infection of cells and the effect of antibodies against viral infection. Calculation results (Formula: anti-viral infection effect = 100 - fluorescence intensity of antibody virus complex group/fluorescence intensity of virus group × 100)

### 3. Results

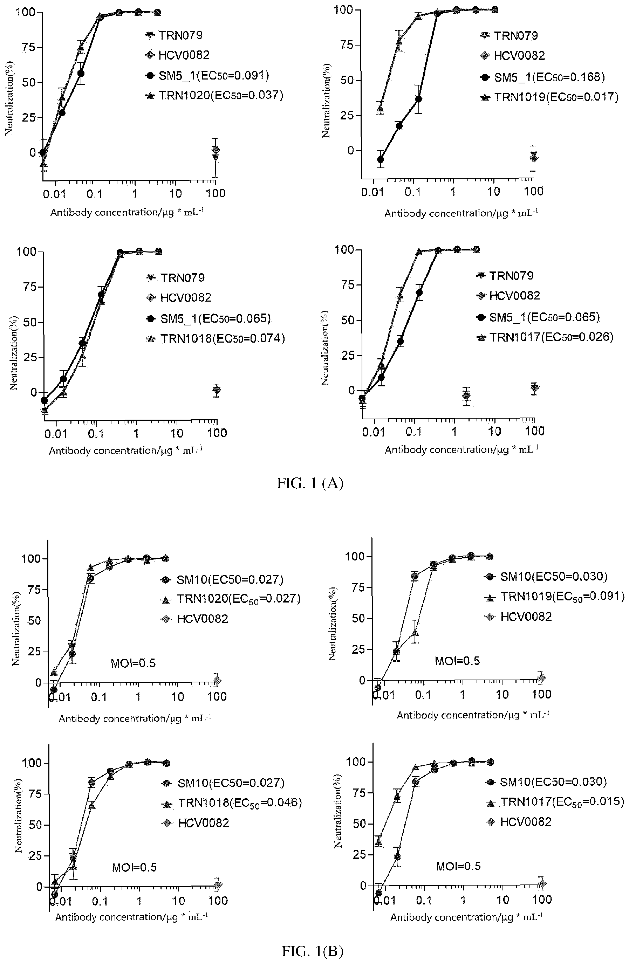

In the results plot, SM5_1 and SM10 are anti-cytomegalovirus antibodies already reported as positive controls; TRNO79 is a rabies virus antibody (CN201611069303.4), HCV0082 is an HCV antibody, and the two unrelated antibodies are used as negative controls. As shown in FIG. 1A, the EC50 of the four strains antibodies TRN1017, TRN1018, TRN1019, and TRN1020 on HCMV standard strain Towne is 0.017-0.074 µg/mL. EC50 refers to half the effective concentration, and refers to the concentration at which 50% of the test animals are induced to produce a specific response, or at which a response index is half inhibited. The smaller EC50 indicates a stronger neutralizing capacity of the antibody. As shown in FIG. 1B, the EC50 of the four strains antibodies TRN1017, TRN1018, TRN1019, and TRN1020 on HCMV clinical strain TB40E is 0.015-0.091 µg/mL. The above results show that the antibodies of the present invention have a very high neutralizing activity against both the standard strain of HCMV Towne, and the strain TB40E.

Meanwhile, two of the antibodies, TRN1017 and TRN1020, were also evaluated in primary HUVEC cell lines for the capability of resisting Towne virus infection, and the antibodies also have extremely high neutralizing activity, with the EC50 being 0.089 µg/mL and 0.041 µg/mL, respectively.

HUVEC is a human umbilical vein endothelial cell and HFF is a human foreskin fibroblast (HFF) cell. The above results demonstrate that the antibodies of the present invention have a higher ability to neutralize virus for different HCMV virus strains; further, the antibodies of the present invention have the ability to neutralize HCMV infected with different cell types, and thus the antibodies of the present invention have the potential to neutralize HCMV at different tissue sites in an individual.

### Example 6 Determination of antibodies affinity

The ability of the antibody to bind to the gB glycoprotein (gB_Towne strain) was determined in vitro using Biacore (SPR principle). The anti-human-IgG (Fc) was coupled to two channels of the CM5 chip by amino coupling, finally channel 1 was coupled with 5485.4RU and channel 2 was coupled with 5622.4RU. The capture antibody concentration was 1 µg/mL and the binding time was 45s. The concentration of bound antigen protein gB0049 was 1.25 µg/mL, 2.5 µg/mL, 5 µg/mL, 10 µg/mL, and 20 µg/mL, with a binding time of 90s, and a dissociation time of 600s. The regeneration solution was 3M MgCl₂ and the regeneration time was 30 s.

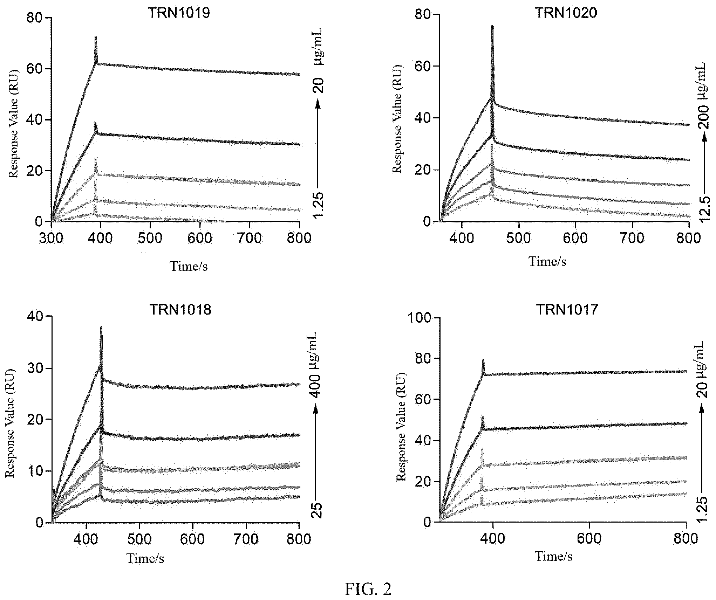

The result was shown in FIG. 2, the K_{D} values of the antibody binding to gB glycoprotein were all on the nM level, wherein the K_{D} value of TRN1020 was 0.60 nM, the K_{D} value of TRN1018 was 27.9 nM, and the K_{D} value of TRN1017 was 1.13 nM, indicating that the antibody of the present invention has a higher binding capacity to the antigen gB glycoprotein.

### Example 7 Studies on antigen-binding epitopes of antibodies

### 1. Effect of antigen deglycosylation on antibody-binding capacity

In the present invention, the effect of gB glycoprotein glycosylation on the antibody was investigated. Firstly, 12 glycosylation sites (N208, N281, N284, N302, N341, N383, N405, N409, N417, N447, N452, and N456) of gB_Towne protein were mutated into glutamine by single point mutation using the kit (the physical and chemical properties of asparagine and glutamine are similar, and the effect of mutation on the spatial structure is minimally changed), then, the binding activity of the mutant of gB glycoprotein to SM10, TRN1017 and TRN1019 antibodies was detected by indirect ELISA, respectively.

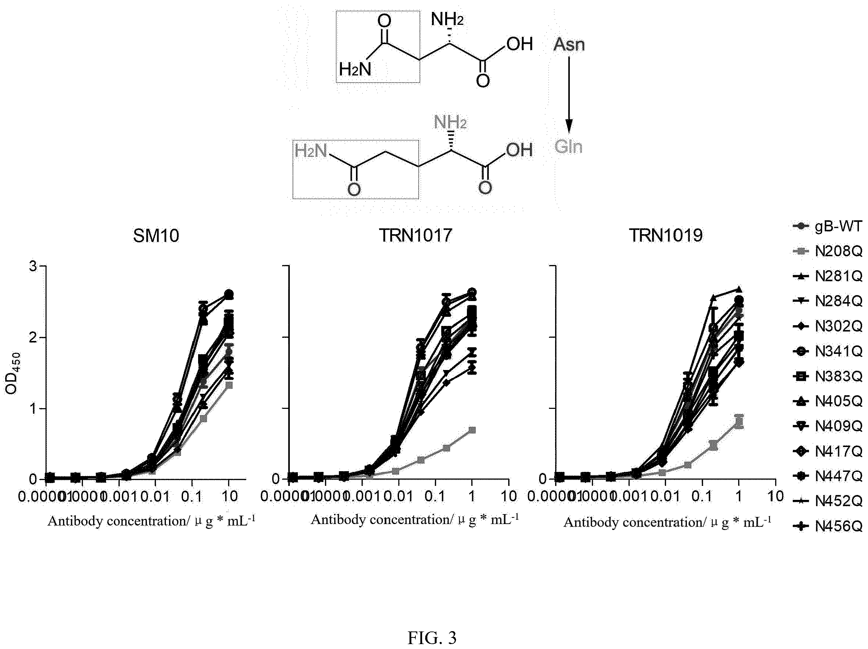

As shown in the results of FIG. 3, after the glycosylation sites of gB glycoproteins N208, N281, N284, N302, N341, N383, N405, N409, N417, N447, N452, and N456 were mutated to glutamine, the binding ability of the TRN1017 and TRN1019 antibodies of the present invention to the N208Q mutant of gB glycoprotein was significantly reduced, and the binding change to other gB glycoprotein mutants was not significant. Meanwhile, considering that the binding epitope of the control antibody SM10 is spatially adjacent to the N208 site, whereas the binding change of SM10 to the gB glycoprotein N208Q mutant is not significant, indicating that the mutation of N208 does not excessively alter the adjacent spatial structure, thus demonstrating the N208 site on the gB glycoprotein to be a key antigen binding epitope for the antibodies of the present invention.

### 3. Site-directed mutation of amino acid site of gB glycoprotein

In order to further identify the epitope information of these antibodies, we performed alanine scanning (186aa-228aa) on the upstream and downstream amino acids centered at N208, and finally screened L213 and Y226 sites. The affinity constants of the TRN1017, TRN1018, TRN1019 and TRN1020 antibodies to the mutants at these two sites were determined using the SPR principle, and the results showed that the binding ability of the antibodies to these two mutants was significantly reduced (no significant change in the binding of the control antibody 1G2), indicating that L213 and Y226 are critical epitopes for the antibodies of the present invention.

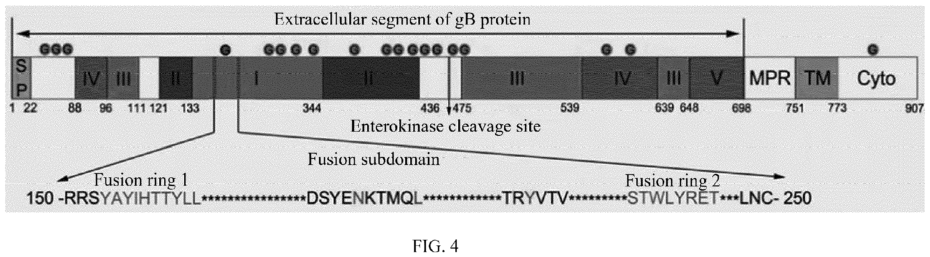

Thus, information on the three epitope amino acids of these antibodies (N208, L213, and Y226) was determined, which are structurally located in the fusion domain of the gB glycoprotein (amino acids R150-C250, which is the structural basis for the membrane fusion function of the gB glycoprotein), and antibodies targeting this domain could directly block virus entry into host cells (FIG. 4).

In conclusion, unlike antibodies that have been reported against AD-1, AD-2, AD-4, and AD-5 domains (Structure of HCMV glycoprotein B in the postfusion conformation bound to a neutralizing human antibody, Sumana Chandramouli et al. Nature Communications volume 6, Article number: 8176 (2015)); Germline V-genes sculpt the binding site of a family of antibodies neutralizing human cytomegalovirus, Christy A Thomson et al. The EMBO Journal (2008) 27, 2592-2602), the antibody epitopes of the present invention are directed against the gB glycoprotein fusion domain. All these antibodies showed high virus neutralization activity and high drug-forming potential. At the same time, the identification of antibody epitope information provides a theoretical basis for the design of HCMV_gB glycoprotein structural vaccine.

### Example 8 Assay for antinuclear resistance of antibody

Hep-2 cell is a human laryngeal cancer epithelial cell, which is internationally generally selected as a substrate for an indirect immunofluorescence method as a detection standard method of the antinuclear antibody because of the characteristics of rich antigen spectrum, strong antigen specificity, and high antigen content of Hep-2. This method was used in the present invention to detect the antinuclear resistance of 4 antibodies of TRN1017, TRN1018, TRN1019, and TRN1020, and the detection was performed according to the instructions of antinuclear antibody (ANA) detection kit.

The results show that the antibodies of TRN1017, TRN1018, TRN1019, and TRN1020 have no immunofluorescence reaction to Hep-2 cells at the concentration of 100 µg/mL, indicating that the antibodies have no autoimmune reaction to Hep-2 cells.

The description of the above Examples is only used to understand the method and core idea of the present invention. It should be noted that, several improvements and modifications may be made by persons of ordinary skill in the art without departing from the principle of the present invention, and these improvements and modifications should also be considered within the protection scope of the present invention.

### Sequence listing:

Listing of heavy chain CDR sequence information

| Serial number | CDR | Sequence information |
|---|---|---|
| SEQ ID NO:1 | CDR1 | GFTFRHYW |
| SEQ ID NO:2 | CDR1 | GFTFSNHG |
| SEQ ID NO:3 | CDR1 | GYTFTNYW |
| SEQ ID NO:4 | CDR1 | GFSFSTYA |
| SEQ ID NO:5 | CDR2 | IHGSGTTT |
| SEQ ID NO:6 | CDR2 | ISSDGTDT |
| SEQ ID NO:7 | CDR2 | IFPRDSYS |
| SEQ ID NO:8 | CDR2 | VSGRGTST |
| SEQ ID NO:9 | CDR3 | VRDDYTSGYN |
| SEQ ID NO:10 | CDR3 | ARDGRCGDERCYSGLPDV |
| SEQ ID NO:11 | CDR3 | AIYNDLRSGNS |
| SEQ ID NO:12 | CDR3 | AKDSYVGGSFDWLPRPDYFDH |

Listing of light chain CDR sequence information

| Serial number | CDR | Sequence information |
|---|---|---|
| SEQ ID NO:21 | CDR1 | RSDVGGYNY |
| SEQ ID NO:22 | CDR1 | QSVGGY |
| SEQ ID NO:23 | CDR1 | QSITKY |
| SEQ ID NO:24 | CDR1 | NSNIGKNY |
| SEQ ID NO:25 | CDR2 | DVS |
| SEQ ID NO:26 | CDR2 | DAS |
| SEQ ID NO:27 | CDR2 | TTS |
| SEQ ID NO:28 | CDR2 | NNN |
| SEQ ID NO:29 | CDR3 | SSYTTKSTLYV |
| SEQ ID NO:30 | CDR3 | QQRSNWPPLT |
| SEQ ID NO:31 | CDR3 | QQSFSTLWT |
| SEQ ID NO:32 | CDR3 | ATWDKTLNFWV |

Listing of amino acid sequence information of antibody heavy chain variable region (VH) and light chain variable region (VL)

| | SEQ ID NO | Variable region | Sequence information |
|---|---|---|---|
| TRN10 17 | SEQ ID NO:13 | VH | QVLLVQSGGGLVQPGGSLRLACETSGFTFRHY WMHWVRQAPGKGLVWLSSIHGSGTTTRYADS VKGRFTISRDNAKNILFLQINSPRPEDTATYYC |
| | | | VRDDYTSGYNWGQGAFVTVSS |
| | SEQ ID NO:33 | VL | QSALTQPASVSGSRGQSITIPCTGSRSDVGGYN YVSWYQQRPGKAPKLILYDVSHRPSGISDRFS GSKLGDTASLTISGLRDEDEADYYCSSYTTKST LYVFGTGTTVSVL |
| TRN10 18 | SEQ ID NO:14 | VH | QVQLVQSGGGVVQPGRSLRLSCAASGFTFSNH GIHWVRQAPGKGLEWVAVISSDGTDTRDADS VRGRFTISRDNSRNTVYLDMNSLRAEDTAVYY CARDGRCGDERCYSGLPDVWGRGTRVSVSS |
| | SEQ ID NO:34 | VL | EIVLTQSPATLSLSPGERATLSCRASQSVGGYLA WYQQKVGQAPRLLIYDASNRATGIPARFSGSG SGTDFTLTISSLEPEDFAVYYCQQRSNWPPLTF GGGTKVEIK |
| TRN10 19 | SEQ ID NO:15 | VH | QVQLVQSGAEVKKPGESLKISCKGSGYTFTNY WIGWVRQMPGAGLEWMAIIFPRDSYSAYSPSF QGRVTISVDKSISTAYLHWSSLEASDTAVYYCA IYNDLRSGNSWGQGTPLIVSS |
| | SEQ ID NO:35 | VL | DIQMTQSPSSLSASVGDRVTITCRASQSITKYL NWYQQKPGRAPKLLIHTTSTLQSGVPSRFSGS GSGTDFTLTISSLQLEDFGTYYCQQSFSTLWTF GQGTKLDIKX |
| TRN10 20 | SEQ ID NO:16 | VH | EVQLVESGGGLAQPGGSLRLSCEASGFSFSTYA MSWVRQAPGKGLEWVAAVSGRGTSTFYLDSV KGRFTVSRDNSRTTVFLQMNSLKVEDTAFYYC AKDSYVGGSFDWLPRPDYFDHWGRGVLVTVS S |
| | SEQ ID NO:36 | VL | QSVLTQPPSLSATPGQTVTISCSGSNSNIGKNY VSWYQQLPGAAPKLLLYNNNLRPSGIPARFSG SKSGTSATLGITGLQAGDEATYSCATWDKTLN |
| | | | FWVFGGGTKVTV |

Amino acid sequences of antibody heavy chain (HC) and light chain (LC)

| | | | |
|---|---|---|---|
| TRN10 17 | SEQ ID NO:37 | HC | QVLLVQSGGGLVQPGGSLRLACETSGFTFRHY WMHWVRQAPGKGLVWLSSIHGSGTTTRYADS VKGRFTISRDNAKNILFLQINSPRPEDTATYYC VRDDYTSGYNWGQGAFVTVSSASTKGPSVFP LAPSSKSTSGGTAALGCLVKDYFPEPVTVSWN SGALTSGVHTFPAVLQSSGLYSLSSVVTVPSSSL GTQTYICNVNHKPSNTKVDKRVEPKSCDKTHT CPPCPAPELLGGPSVFLFPPKPKDTLMISRTPEV TCVVVDVSHEDPEVKFNWYVDGVEVHNAKT KPREEQYNSTYRVVSVLTVLHQDWLNGKEYK CKVSNKALPAPIEKTISKAKGQPREPQVYTLPP SREEMTKNQVSLTCLVKGFYPSDIAVEWESNG QPENNYKTTPPVLDSDGSFFLYSKLTVDKSRW QQGNVFSCSVMHEALHNHYTQKSLSLSPGK |
| | SEQ ID NO:38 | LC | QSALTQPASVSGSRGQSITIPCTGSRSDVGGYN YVSWYQQRPGKAPKLILYDVSHRPSGISDRFS GSKLGDTASLTISGLRDEDEADYYCSSYTTKST LYVFGTGTTVSVLGQPKAAPSVTLFPPSSEELQ ANKATLVCLISDFYPGAVTVAWKADSSPVKAG VETTTPSKQSNNKYAASSYLSLTPEQWKSHRS YSCQVTHEGSTVEKTVAPTECS |
| TRN10 18 | SEQ ID NO:39 | HC | QVQLVQSGGGVVQPGRSLRLSCAASGFTFSNH GIHWVRQAPGKGLEWVAVISSDGTDTRDADS VRGRFTISRDNSRNTVYLDMNSLRAEDTAVYY CARDGRCGDERCYSGLPDVWGRGTRVSVSSA STKGPSVFPLAPSSKSTSGGTAALGCLVKDYFP EPVTVSWNSGALTSGVHTFPAVLQSSGLYSLSS |
| | | | VVTVPSSSLGTQTYICNVNHKPSNTKVDKRVE PKSCDKTHTCPPCPAPELLGGPSVFLFPPKPKD TLMISRTPEVTCVVVDVSHEDPEVKFNWYVD GVEVHNAKTKPREEQYNSTYRVVSVLTVLHQ DWLNGKEYKCKVSNKALPAPIEKTISKAKGQP REPQVYTLPPSREEMTKNQVSLTCLVKGFYPS DIAVEWESNGQPENNYKTTPPVLDSDGSFFLY SKLTVDKSRWQQGNVFSCSVMHEALHNHYTQ KSLSLSPGK |
| | SEQ ID NO:40 | LC | EIVLTQSPATLSLSPGERATLSCRASQSVGGYLA WYQQKVGQAPRLLIYDASNRATGIPARFSGSG SGTDFTLTISSLEPEDFAVYYCQQRSNWPPLTF GGGTKVEIKRTVAAPSVFIFPPSDEQLKSGTAS VVCLLNNFYPREAKVQWKVDNALQSGNSQES VTEQDSKDSTYSLSSTLTLSKADYEKHKVYAC EVTHQGLSSPVTKSFNRGEC |
| TRN10 19 | SEQ ID NO:41 | HC | QVQLVQSGAEVKKPGESLKISCKGSGYTFTNY WIGWVRQMPGAGLEWMAIIFPRDSYSAYSPSF QGRVTISVDKSISTAYLHWSSLEASDTAVYYCA IYNDLRSGNSWGQGTPLIVSSASTKGPSVFPLA PSSKSTSGGTAALGCLVKDYFPEPVTVSWNSG ALTSGVHTFPAVLQSSGLYSLSSVVTVPSSSLGT QTYICNVNHKPSNTKVDKRVEPKSCDKTHTCP PCPAPELLGGPSVFLFPPKPKDTLMISRTPEVTC VVVDVSHEDPEVKFNWYVDGVEVHNAKTKP REEQYNSTYRVVSVLTVLHQDWLNGKEYKCK VSNKALPAPIEKTISKAKGQPREPQVYTLPPSR EEMTKNQVSLTCLVKGFYPSDIAVEWESNGQP ENNYKTTPPVLDSDGSFFLYSKLTVDKSRWQQ |
| | | | GNVFSCSVMHEALHNHYTQKSLSLSPGK |
| | SEQ ID NO:42 | LC | DIQMTQSPSSLSASVGDRVTITCRASQSITKYL NWYQQKPGRAPKLLIHTTSTLQSGVPSRFSGS GSGTDFTLTISSLQLEDFGTYYCQQSFSTLWTF GQGTKLDIKRTVAAPSVFIFPPSDEQLKSGTAS VVCLLNNFYPREAKVQWKVDNALQSGNSQES VTEQDSKDSTYSLSSTLTLSKADYEKHKVYAC EVTHQGLSSPVTKSFNRGEC |
| TRN10 20 | SEQ ID NO:43 | HC | EVQLVESGGGLAQPGGSLRLSCEASGFSFSTYA MSWVRQAPGKGLEWVAAVSGRGTSTFYLDSV KGRFTVSRDNSRTTVFLQMNSLKVEDTAFYYC AKDSYVGGSFDWLPRPDYFDHWGRGVLVTVS SASTKGPSVFPLAPSSKSTSGGTAALGCLVKDY FPEPVTVSWNSGALTSGVHTFPAVLQSSGLYSL SSVVTVPSSSLGTQTYICNVNHKPSNTKVDKR VEPKSCDKTHTCPPCPAPELLGGPSVFLFPPKP KDTLMISRTPEVTCVVVDVSHEDPEVKFNWY VDGVEVHNAKTKPREEQYNSTYRVVSVLTVL HQDWLNGKEYKCKVSNKALPAPIEKTISKAK GQPREPQVYTLPPSREEMTKNQVSLTCLVKGF YPSDIAVEWESNGQPENNYKTTPPVLDSDGSF FLYSKLTVDKSRWQQGNVFSCSVMHEALHNH YTQKSLSLSPGK |
| | SEQ ID NO:44 | LC | QSVLTQPPSLSATPGQTVTISCSGSNSNIGKNY VSWYQQLPGAAPKLLLYNNNLRPSGIPARFSG SKSGTSATLGITGLQAGDEATYSCATWDKTLN FWVFGGGTKVTVLGQPKAAPSVTLFPPSSEEL QANKATLVCLISDFYPGAVTVAWKADSSPVKA GVETTTPSKQSNNKYAASSYLSLTPEQWKSHR |
| | | | SYSCQVTHEGSTVEKTVAPTECS |

Light chain λ constant region: 
GQPKAAPSVTLFPPSSEELQANKATLVCLISDFYPGAVTVAWKADSSPVKAGVETTTPSKQ
SNNKYAASSYLSLTPEQWKSHRSYSCQVTHEGSTVEKTVAPTECS （SEQ ID NO:17）
Light chain κ constant region: 

 （SEQ ID NO:18）
Heavy chain constant region:

ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSKLTVDKSRWQQG
NVFSCSVMHEALHNHYTQKSLSLSPGK（SEQ ID NO:45）

## Claims

1. A monoclonal antibody or an antigen-binding fragment thereof specifically binding to human cytomegalovirus gB glycoprotein, comprising a heavy chain variable region (VH) and a light chain variable region (VL), wherein the VH comprises three complementarity determining regions (HCDRs) of the heavy chain variable region shown in SEQ ID NO: 13, 14, 15, or 16, and the VL comprises three complementarity determining regions (LCDRs) of the light chain variable region shown in SEQ ID NO: 33, 34, 35, or 36.

2. The antibody or antigen-binding fragment thereof according to claim 1, comprising:
(1) three CDRs of the heavy chain variable region shown in SEQ ID NO: 13, and three CDRs of the light chain variable region shown in SEQ ID NO: 33;
(2) three CDRs of the heavy chain variable region shown in SEQ ID NO: 14, and three CDRs of the light chain variable region shown in SEQ ID NO: 34;
(3) three CDRs of the heavy chain variable region shown in SEQ ID NO: 15, and three CDRs of the light chain variable region shown in SEQ ID NO: 35;
(4) three CDRs of the heavy chain variable region shown in SEQ ID NO: 16, and three CDRs of the light chain variable region shown in SEQ ID NO: 36.

3. The antibody or antigen-binding fragment thereof according to claim 1, comprising a heavy chain variable region (VH) and a light chain variable region (VL), wherein
(i) the VH comprises the complementarity determining regions (CDR) HCDR1, HCDR2, and HCDR3, wherein HCDR1 comprises or consists of the amino acid sequence of SEQ ID NO: 1, 2, 3, or 4; HCDR2 comprises or consists of the amino acid sequence of SEQ ID NO: 5, 6, 7, or 8; HCDR3 comprises or consists of the amino acid sequence of SEQ ID NO: 9, 10, 11, or 12;
and,
(ii) wherein the VL comprises the complementarity determining regions (CDR) LCDR1, LCDR2, and LCDR3, wherein LCDR1 comprises or consists of the amino acid sequence of SEQ ID NO: 21, 22, 23, or 24; LCDR2 comprises or consists of the amino acid sequence of SEQ ID NO: 25, 26, 27, or 28; LCDR3 comprises or consists of the amino acid sequence of SEQ ID NO: 29, 30, 31, or 32.

4. The antibody or antigen-binding fragment thereof according to claim 1, comprising a heavy chain variable region (VH) and a light chain variable region (VL), wherein
(i) the VH comprises HCDR1, HCDR2, and HCDR3 comprising or consisting of the following sequences of SEQ ID NO: 1, SEQ IDNO: 5, and SEQ ID NO: 9; and the VL comprises LCDR1, LCDR2, and LCDR3 comprising or consisting of the following sequences of SEQ ID NO: 21, SEQ IDNO: 25, and SEQ ID NO: 29; or
(ii) the VH comprises HCDR1, HCDR2, and HCDR3 comprising or consisting of the following sequences of SEQ ID NO: 2, SEQ IDNO: 6, and SEQ ID NO: 10; and the VL comprises LCDR1, LCDR2, and LCDR3 comprising or consisting of the following sequences of SEQ ID NO: 22, SEQ IDNO: 26, and SEQ ID NO: 30; or
(iii) the VH comprises HCDR1, HCDR2, and HCDR3 comprising or consisting of the following sequences of SEQ ID NO: 3, SEQ IDNO: 7, and SEQ ID NO: 11; and the VL comprises LCDR1, LCDR2, and LCDR3 comprising or consisting of the following sequences of SEQ ID NO: 23, SEQ IDNO: 27, and SEQ ID NO: 31; or
(iv) the VH comprises HCDR1, HCDR2, and HCDR3 comprising or consisting of the following sequences of SEQ ID NO: 4, SEQ IDNO: 8, and SEQ ID NO: 12; and the VL comprises LCDR1, LCDR2, and LCDR3 comprising or consisting of the following sequences of SEQ ID NO: 24, SEQ IDNO: 28, and SEQ ID NO: 32.

5. An antibody or an antigen-binding fragment thereof specifically binding to human cytomegalovirus gB glycoprotein, comprising a heavy chain variable region VH and a light chain variable region VL, wherein
(a) the heavy chain variable region VH
(i) comprises or consists of an amino acid sequence that is at least 90%, 91%, 92%, 93%, 94%, 95%, 96%, 97%, 98%, or 99% identical to an amino acid sequence selected from the group consisting of SEQ ID NOs: 13, 14, 15, or 16; or
(ii) comprises or consists of an amino acid sequence selected from SEQ ID NO: 13, 14, 15, or 16; or
(iii) comprises an amino acid sequence having 1 or more (preferably no more than 10, more preferably no more than 5, 4, 3, 2, 1) amino acid changes (preferably amino acid substitutions, more preferably amino acid conservative substitutions) compared to an amino acid sequence selected from SEQ ID NO: 13, 14, 15 or 16, wherein the amino acid changes do not occur in the CDR regions;
and,
(b) the light chain variable region VL
(i) comprises or consists of an amino acid sequence that is at least 90%, 91%, 92%, 93%, 94%, 95%, 96%, 97%, 98%, or 99% identical to an amino acid sequence selected from the group consisting of SEQ ID NO: 33, 34, 35, or 36;
(ii) comprises or consists of an amino acid sequence selected from SEQ ID NO: 33, 34, 35, or 36; or
(iii) comprises an amino acid sequence having 1 or more (preferably no more than 10, more preferably no more than 5, 4, 3, 2, 1) amino acid changes (preferably amino acid substitutions, more preferably amino acid conservative substitutions) compared to an amino acid sequence selected from SEQ ID NO 33, 34, 35 or 36, wherein the amino acid changes do not occur in the CDR regions.

6. The antibody or antigen-binding fragment thereof according to claim 5, wherein
(i) the VH comprises or consists of the amino acid sequence of SEQ ID NO: 13: the VL comprises or consists of the amino acid sequence of SEQ ID NO: 33;2
(ii) the VH comprises or consists of the amino acid sequence of SEQ ID NO: 14; the VL comprises or consists of the amino acid sequence of SEQ ID NO: 34;
(iii) the VH comprises or consists of the amino acid sequence of SEQ ID NO: 15; the VL comprises or consists of the amino acid sequence of SEQ ID NO: 35; or
(iv) the VH comprises or consists of the amino acid sequence of SEQ ID NO: 16; and the VL comprises or consists of the amino acid sequence of SEQ ID NO: 36.

7. An antibody or an antigen-binding fragment thereof specifically binding to human cytomegalovirus gB glycoprotein, comprising a heavy chain and a light chain, wherein
(a) the heavy chain
(i) comprises or consists of an amino acid sequence that is at least 85%, 90%, 91%, 92%, 93%, 94%, 95%, 96%, 97%, 98%, or 99% identical to an amino acid sequence selected from the group consisting of SEQ ID NOs: 37, 39, 41, or 43; or
(ii) comprises or consists of an amino acid sequence selected from SEQ ID NO: 37, 39, 41, or 43; or
(iii)comprises an amino acid sequence having 1 or more (preferably no more than 20 or 10, more preferably no more than 5, 4, 3, 2, 1) amino acid changes(preferably amino acid substitutions, more preferably amino acid conservative substitutions) compared to an amino acid sequence selected from SEQ ID NO 37, 39, 41 or 43, preferably the amino acid changes do not occur in the CDR regions of the heavy chain, more preferably the amino acid changes do not occur in the variable region of the heavy chain;
and/or
(b) the light chain
(i) comprises or consists of an amino acid sequence that is at least 85%, 90%, 91%, 92%, 93%, 94%, 95%, 96%, 97%, 98%, or 99% identical to an amino acid sequence selected from the group consisting of SEQ ID NOs: 38, 40, 42, or 44; or
(ii) comprises or consists of an amino acid sequence selected from SEQ ID NO: 38, 40, 42, or 44; or
(iii)comprises an amino acid sequence having 1 or more (preferably no more than 20 or 10, more preferably no more than 5, 4, 3, 2, 1) amino acid changes(preferably amino acid substitutions, more preferably amino acid conservative substitutions) compared to an amino acid sequence selected from SEQ ID NO 38, 40, 42 or 44, preferably the amino acid changes do not occur in the CDR regions of the light chain, more preferably the amino acid changes do not occur in the variable region of the light chain.

8. An antibody or an antigen-binding fragment thereof specifically binding to human cytomegalovirus gB glycoprotein, wherein the antibody or antigen-binding fragment thereof binds to an epitope on the gB glycoprotein fusion domain comprising one or more amino acid residues selected from the group consisting of N208, L213, and Y226 of the gB glycoprotein fusion domain.

9. The antibody or antigen-binding fragment thereof according to claim 8, comprising one, two, or all three CDRs in the HCDR of the heavy chain variable region shown in SEQ ID NO: 13 or SEQ ID NO: 15, and/or one, two, or all three CDRs in the LCDR of the light chain variable region shown in SEQ ID NO: 33 or SEQ ID NO: 35.

10. The antibody or antigen-binding fragment thereof according to claim 8, comprising one, two, or all three CDRs in HCDR1 shown in SEQ ID NO: 1 or 3, HCDR2 shown in SEQ ID NO: 5 or 7, HCDR3 shown in SEQ ID NO: 9 or 11 and/or one, two, or all three CDRs in HCDR1 shown in SEQ ID NO: 21 or 23, HCDR2 shown in SEQ ID NO: 25 or 27, HCDR3 shown in SEQ ID NO: 29 or 31.

11. The antibody or antigen-binding fragment thereof according to claim 8, comprising the heavy chain variable region shown in SEQ ID NO: 13 or SEQ ID NO: 15, and/or the light chain variable region shown in SEQ ID NO: 33 or SEQ ID NO: 35.

12. The antibody or antigen-binding fragment thereof according to any one of claims 1-11, wherein the antibody or antigen-binding fragment thereof binds to human cytomegalovirus gB glycoprotein with a K_{D} of about 50 nM or less.

13. The antibody or antigen-binding fragment thereof according to any one of claims 1-11, wherein the antibody or antigen-binding fragment thereof is capable of binding to the fusion domain of the human cytomegalovirus gB glycoprotein.

14. The antibody or antigen-binding fragment thereof according to any one of claims 1-11, wherein at least a portion of the framework sequences are a human consensus framework sequences.

15. The antibody or antigen-binding fragment thereof of any one of claims 1-11, wherein the antibody is a human monoclonal antibody.

16. The antibody or antigen-binding fragment thereof according to any one of claims 1-11, wherein the antigen-binding fragment is selected from the group consisting of Fab, Fab', Fab'-SH, Fv, a single chain antibody (e.g., Scfv) or (Fab')₂, a single domain antibody, a diabody (dAb), or a linear antibody.

17. The antibody according to any of the preceding claims, wherein the antibody is an IgG-like antibody, such as an antibody in the form of IgG1 or an antibody in the form of IgG2 or an antibody in the form of IgG3 or an antibody in the form of IgG4.

18. An isolated nucleic acid encoding the antibody or antigen-binding fragment thereof according to any one of claims 1-17.

19. A vector comprising the nucleic acid according to claim 18, the vector being an expression vector.

20. A host cell comprising a vector according to claim 19, wherein the host cell is a eukaryotic or prokaryotic cell, such as an E. Coli cell, a yeast cell, a mammalian cell, or a plant cell, preferably the host cell is a CHO cell or a 293 cell, such as a HEK293 cell.

21. A method of producing the antibody or antigen-binding fragment thereof according to any one of the preceding claims, the method comprising culturing the host cell comprising the nucleic acid according to claim 18 or the expression vector according to claim 19.

22. The method according to claim 21, wherein the method further comprises recovering the antibody or antigen-binding fragment thereof from the host cell or culture medium.

23. An immunoconjugate comprising the antibody or antigen-binding fragment thereof according to any one of claims 1-17 and a label.

24. A pharmaceutical composition comprising the antibody or antigen-binding fragment thereof according to any one of claims 1-17 or the immunoconjugate according to claim 23, and optionally one or more pharmaceutically acceptable auxiliary materials, such as a pharmaceutically acceptable carrier, a pharmaceutically acceptable excipient, including a buffer or diluent.

25. Use of the antibody or antigen-binding fragment thereof according to any one of claims 1-17 or the immunoconjugate according to claim 23 in the preparation of a pharmaceutical composition or kit for the detection, treatment, prevention and/or alleviation of a HCMV infection or a HCMV-related disease.

26. A method of preventing or treating a HCMV infection or a HCMV-related disease in a human subject, comprising administering to a subject in need of such treatment an effective amount of the antibody or antigen-binding fragment thereof according to any one of claims 1-17, or the immunoconjugate according to claim 23, or the pharmaceutical composition according to claim 24.

27. A method of increasing, enhancing, or stimulating resistance in a human subject infected with HCMV, comprising administering to a subject in need thereof an effective amount of the antibody or antigen-binding fragment thereof according to any one of claims 1-17, or the immunoconjugate according to claim 23, or the pharmaceutical composition according to claim 24.

28. The method according to claim 26 or 27, wherein the subject is a HCMV-infected subject.

29. The method according to claim 26 or 27, wherein the subject is a transplant patient, a pregnant woman, a newborn, an AIDS patient, a cancer patient, or a patient with autoimmune diseases.

30. A method of neutralizing HCMV in an individual or sample, comprising contacting the antibody or antigen-binding fragment thereof according to any one of claims 1-17 with the individual or sample and testing the capacity of the antibody or antigen-binding fragment thereof to bind to neutralize HCMV.
